# EUROPEAN PATENT SPECIFICATION

(11) **EP 2 895 624 B1**
(45) Date of publication and mention of the grant of the patent: **01.06.2022**
(21) Application number: 13837233.9
(22) Date of filing: 13.09.2013
(51) Int. Cl.: C12Q 1/6883

(54) **METHODS OF PREDICTING THE DEVELOPMENT OF AMD BASED ON CHROMOSOME 1 AND CHROMOSOME 10**
VERFAHREN ZUR VORHERSAGE DER ENTWICKLUNG VON AMD AUF DER BASIS VON CHROMOSOM 1 UND CHROMOSOM 10
MÉTHODES DE PRÉDICTION DU DÉVELOPPEMENT D'UNE DMA SUR LA BASE DU CHROMOSOME 1 ET DU CHROMOSOME 10

(30) Priority: 14.09.2012 US 201261701322 P
(43) Date of publication of application: 22.07.2015
(73) Proprietor: University of Utah Research Foundation, Salt Lake City, UT 84108 (US)
(72) Inventor: HAGEMAN, Gregory, Salt Lake City, UT 84132 (US); PAPPAS, Christian, Matthew, Salt Lake City, UT 84105 (US)
(74) Representative: Hoffmann Eitle
(86) International application number: PCT/US2013/059789
(87) International publication number: WO 2014/043558

(56) References cited:
- WO-A2-2006/088950
- WO-A2-2006/096737
- WO-A2-2008/013893
- WO-A2-2008/140793
- WO-A2-2012/051462
- US-A1- 2010 330 097
- US-A1- 2012 142 608
- HAGEMAN G S ET AL: "A common haplotype in the complement regulatory gene factor H (HF1/CFH) predisposes individuals to age-related macular degeneration", PROCEEDINGS OF THE NATIONAL ACADEMY OF SCIENCES, NATIONAL ACADEMY OF SCIENCES, US, vol. 102, no. 20, 17 May 2005 (2005-05-17) , pages 7227-7232, XP002544686, ISSN: 0027-8424, DOI: 10.1073/PNAS.051536102 [retrieved on 2006-02-08]

### SEQUENCE LISTING

<110> Hageman, Gregory Pappas, Christian M
<120> METHODS OF PREDICTING THE DEVELOPMENT OF AMD BASED ON CHROMOSOME 1 AND CHROMOSOME 10
<130> 21101.0274P1
<150> 61/701,322
   <151> 2012-09-14
<160> 5
<170> PatentIn version 3.5
<210> 1
   <211> 51
   <212> DNA
   <213> Homo sapiens
<220>
   <221> misc_feature
   <222> (1)..(51)
   <223> chromosome 1 complement factor H haplotype H1
<400> 1
   accgatcaac catgacaagg ccgccggacg cggggttgcg cggcttcgtc c 51
<210> 2
   <211> 51
   <212> DNA
   <213> Homo sapiens
<220>
   <221> misc_feature
   <222> (1)..(51)
   <223> chromosome 1 complement factor H haplotype H2
<400> 2
   accgatcaac catgacaagg ccgccggacg cggggttgcg tatcttcatc c 51
<210> 3
   <211> 51
   <212> DNA
   <213> Homo sapiens
<220>
   <221> misc_feature
   <222> (1)..(51)
   <223> chromosome 1 complement factor H haplotype H3
<400> 3
   actaatcctt ttcataagag tcgccggata cggggttgct cgttttcgtc c 51
<210> 4
   <211> 51
   <212> DNA
   <213> Homo sapiens
<220>
   <221> misc_feature
   <222> (1)..(51)
   <223> chromosome 1 complement factor H haplotype H4
<400> 4
   atcgatcctt tacgaagggg ccttcgaatg caaagtagat tatcttcatc c 51
<210> 5
   <211> 51
   <212> DNA
   <213> Homo sapiens
<220>
   <221> misc_feature
   <222> (1)..(51)
   <223> chromosome 1 complement factor H haplotype H5
<400> 5
   accggctctt tatataagat ccgccggatg caggtctgat catctttact t 51

## Description

### BACKGROUND

Age related macular degeneration (AMD), is the leading cause of blindness in the elderly, a problem that will grow worse with an aging population. Accurate identification of risk for developing complement-mediated diseases such as AMD is essential for both starting treatment early in disease progression and identifying causal genetic variants to better target therapeutic strategies and development. Previously, loci have been implicated in risk for AMD on Chr1, Chr10, Chr6 (CFB), and Chr19 (C3).

Previously, studies relating to AMD and the presence of single nucleotide polymorphisms (SNPs), haplotypes, and diplotypes have focused on genes located on a variety of chromosomes. What is needed are more effective methods of describing risk for having or developing AMD based on genes located on chromosome 1 and chromosome 10. Additionally, what is needed are methods of appropriately enrolling patients in AMD clinical trials based on their genetic profile at chromosome 1 and chromosome 10.

### SUMMARY

Described herein is a method as defined by the appended claims for determining a subject's susceptibility to having or developing AMD comprising determining in the subject the identity of one or more chromosome 1 and chromosome 10 SNPs, haplotypes, or diplotypes described herein.

### BRIEF DESCRIPTION OF THE FIGURES

The accompanying figures, which are incorporated in and constitute a part of this specification, illustrate several aspects and together with the description serve to explain the principles of the invention.
Figure 1 shows a summary of chromosome 1 diplotype and chromosome 10 genotype vs. AMD phenotype.

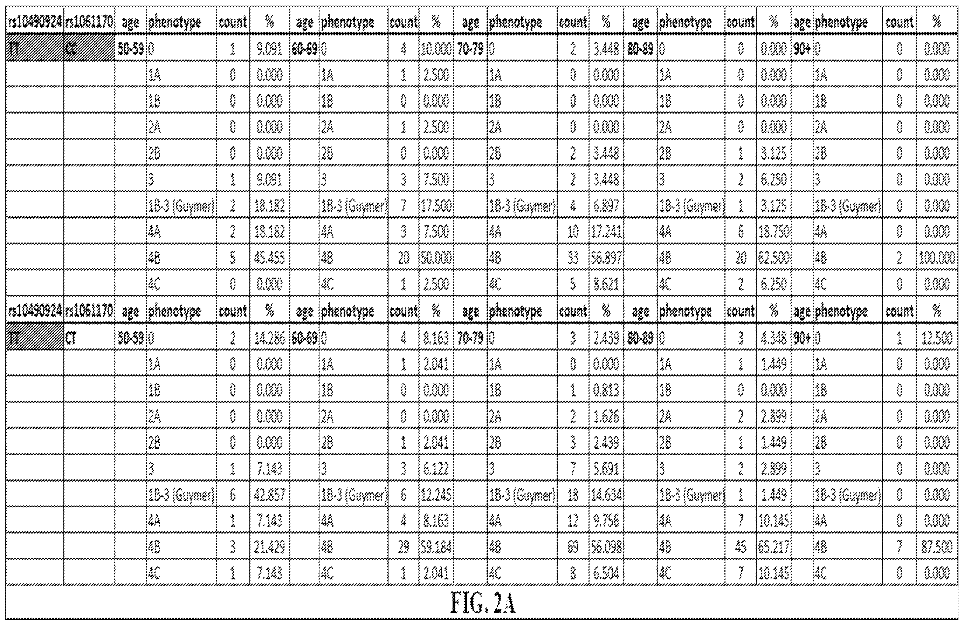

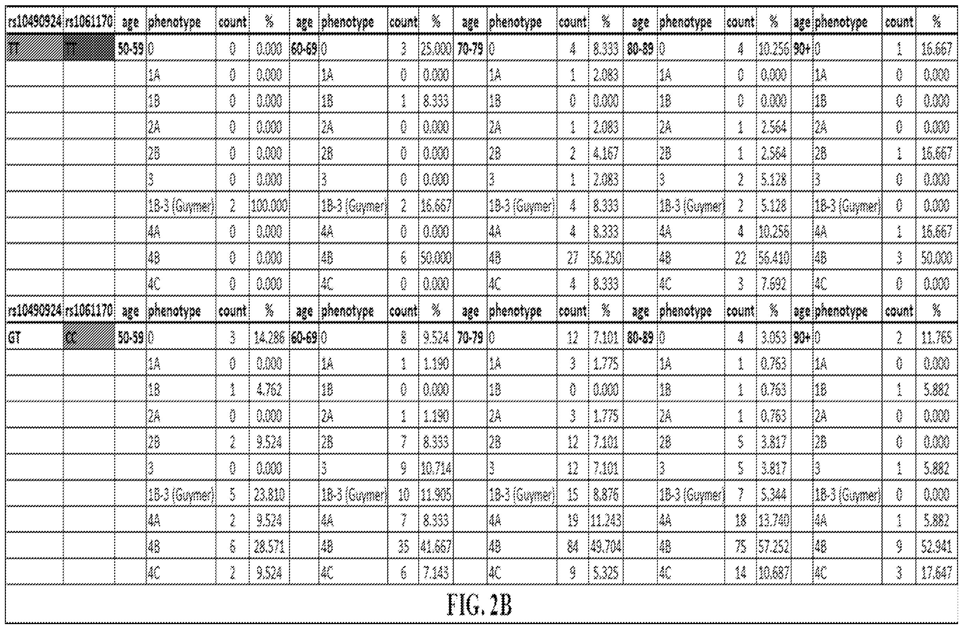

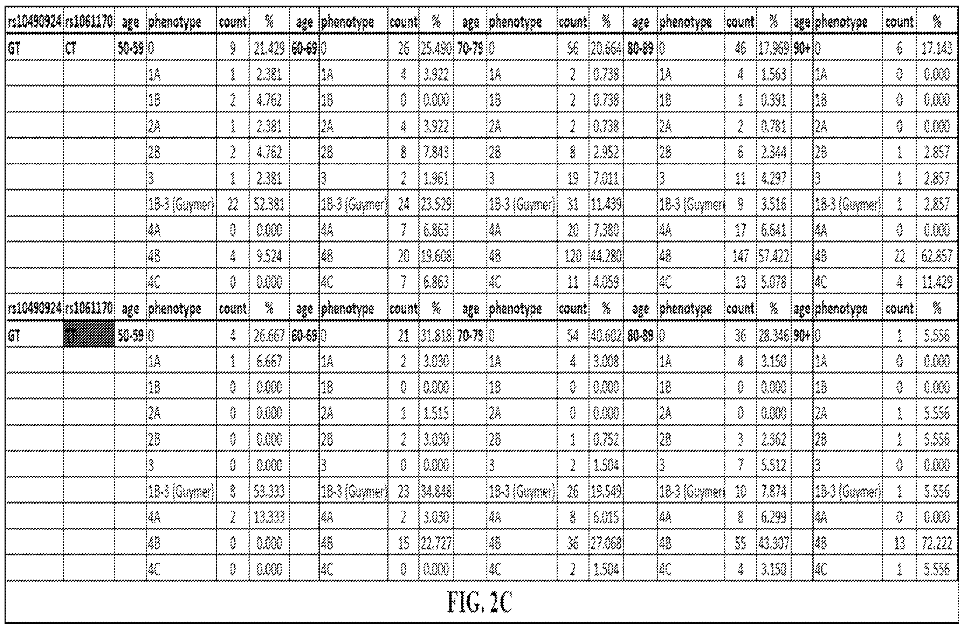

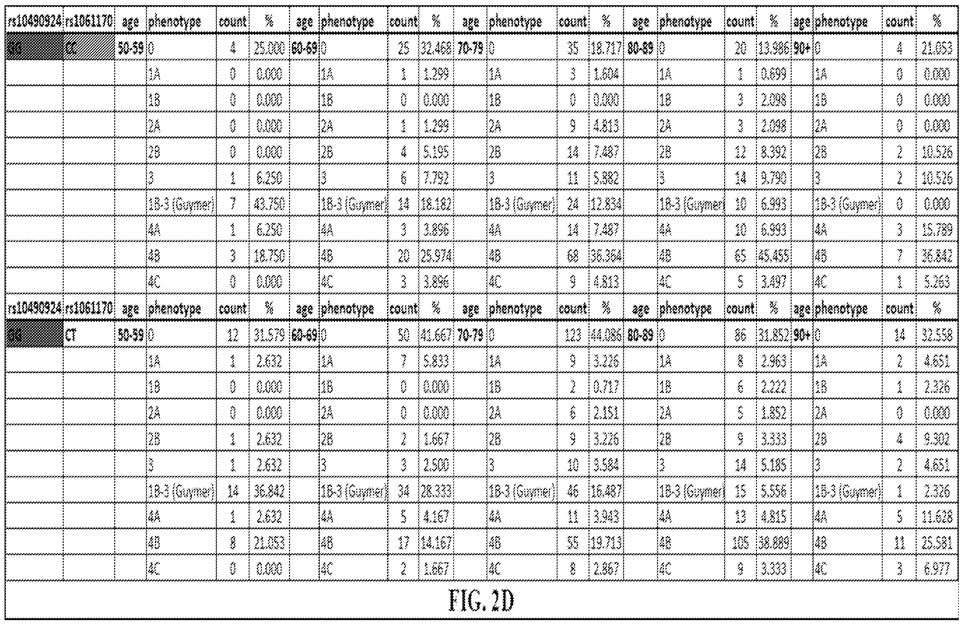

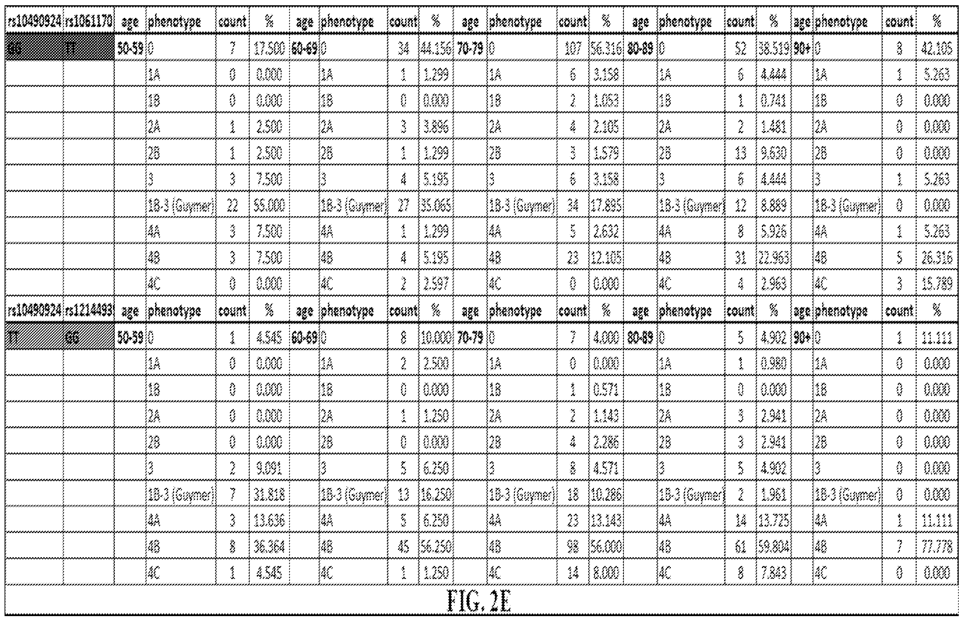

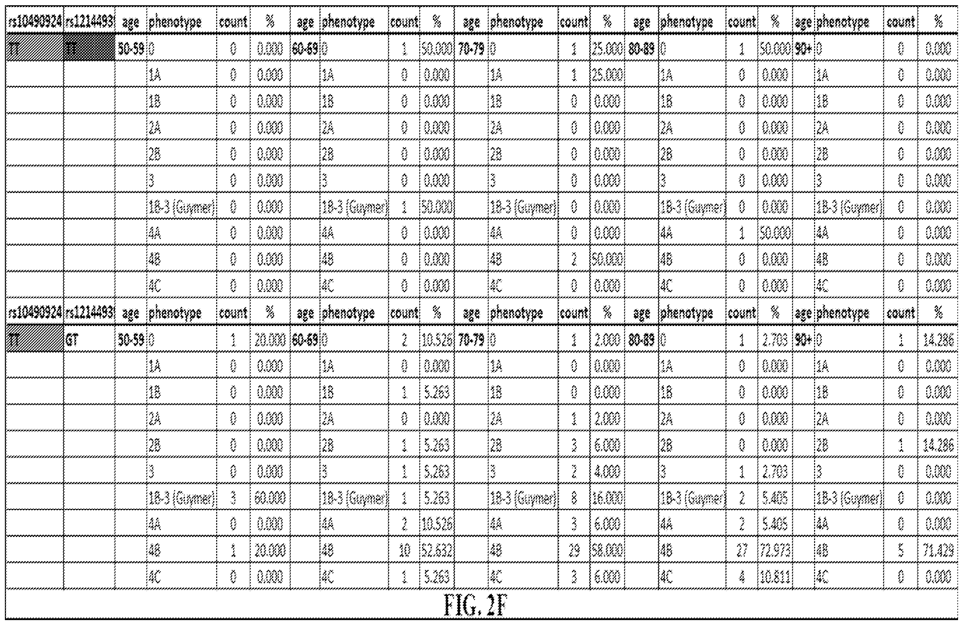

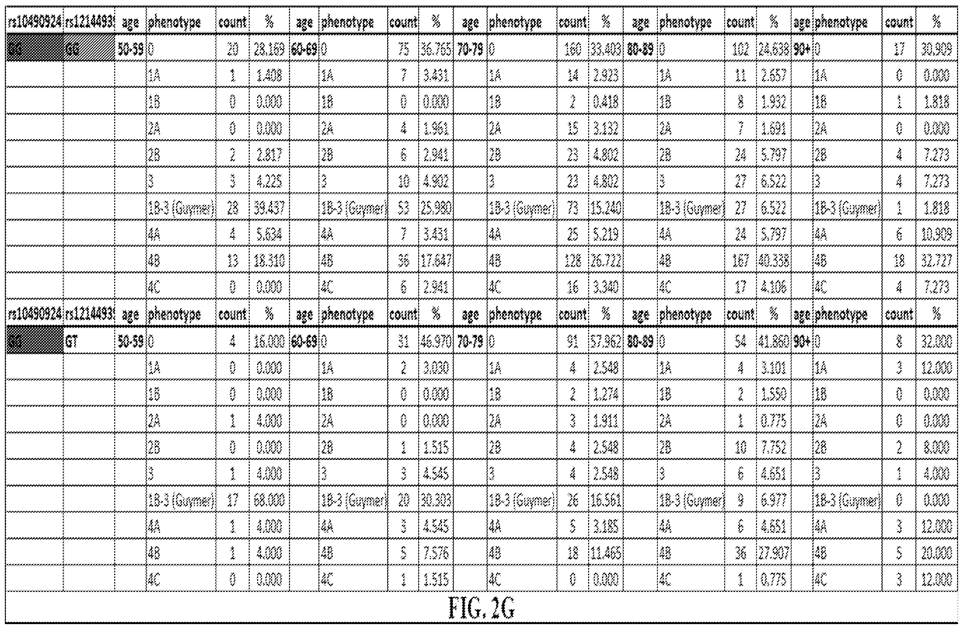

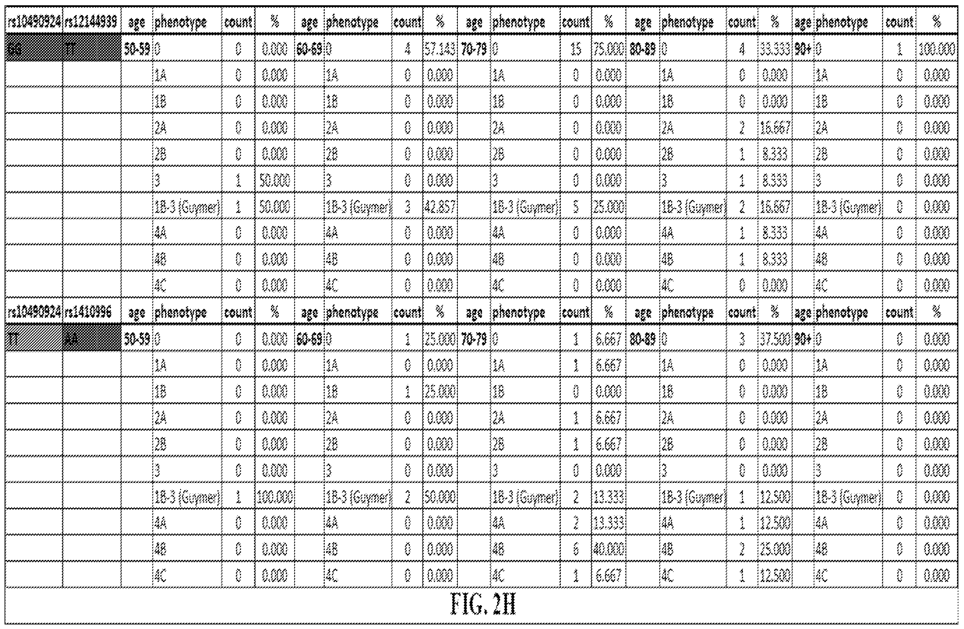

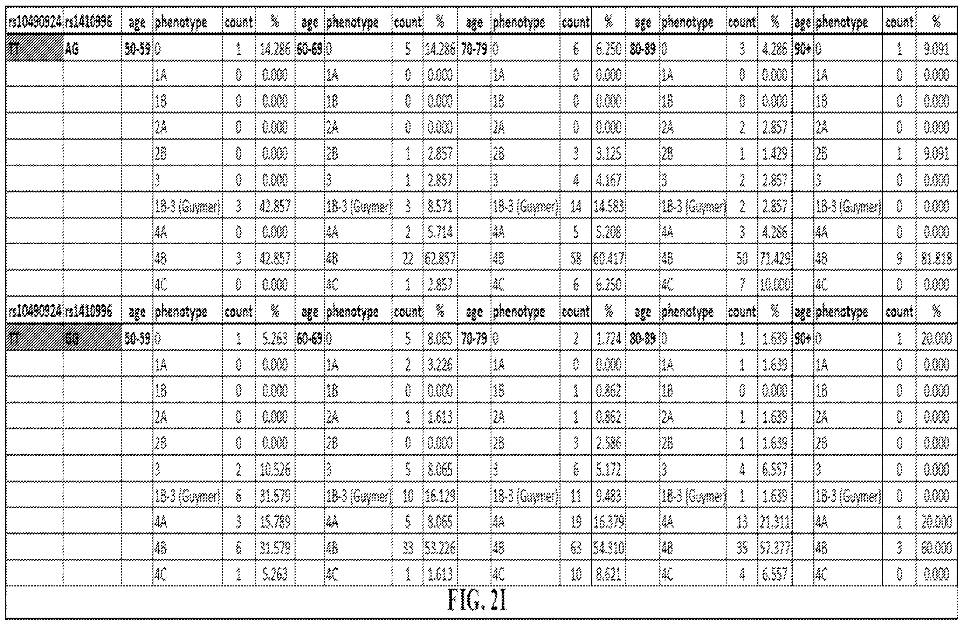

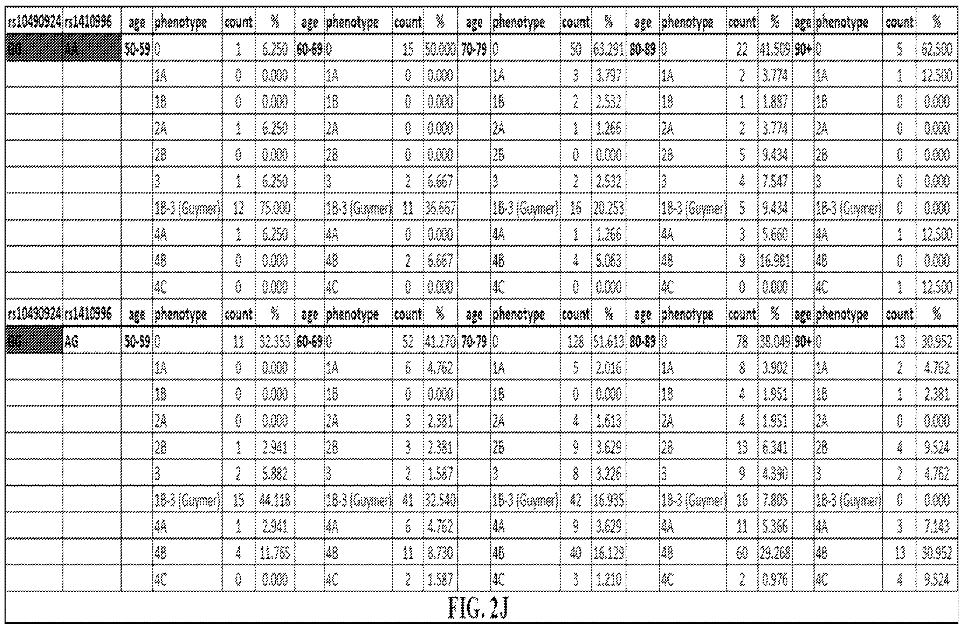

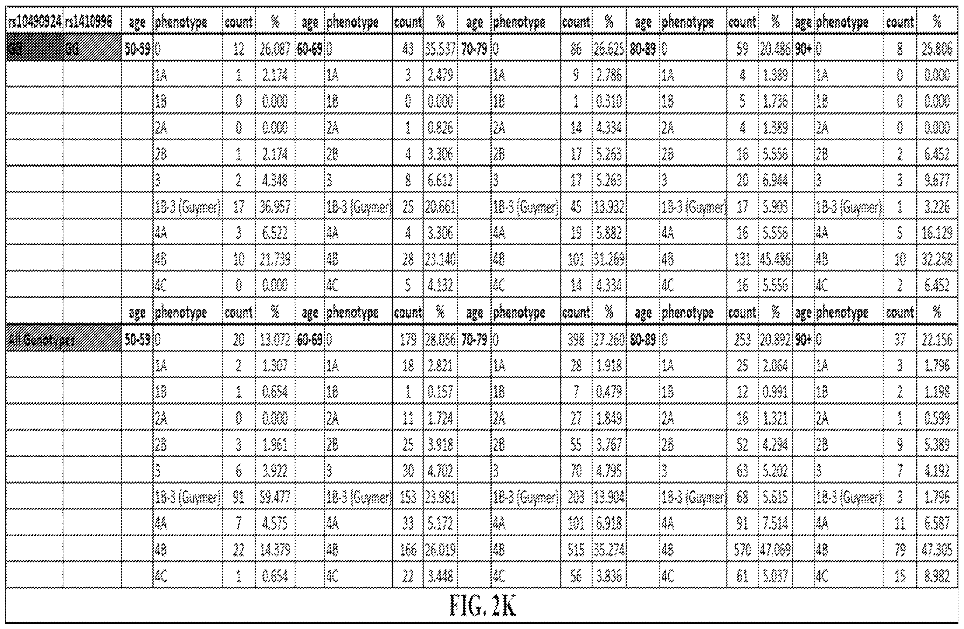

Figure 2 shows a breakdown of chromosome 1 and chromosome 10 genotype and the relation to AMD phenotype based on the age of the subject.

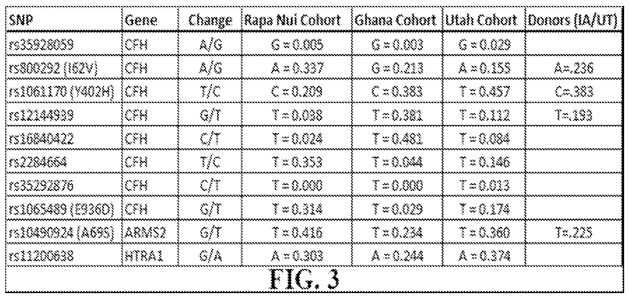

Figure 3 shows the frequency of various chromosome 1 and chromosome 10 alleles in different ethnic populations.

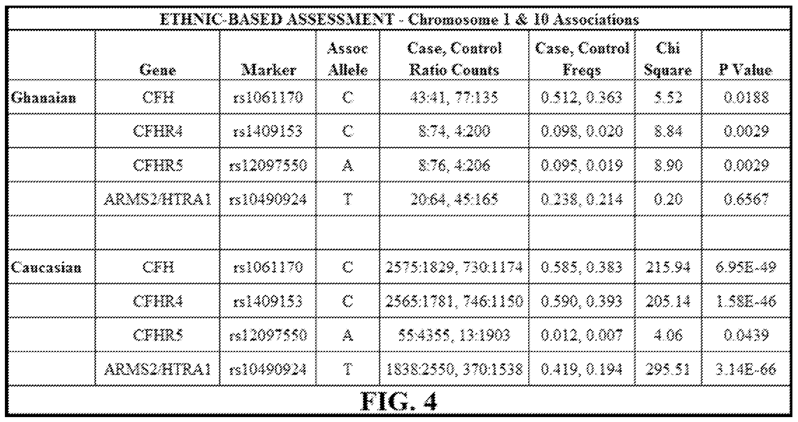

Figure 4 shows chromosome 1 and chromosome 10 genotype associations with AMD in different ethnic populations.

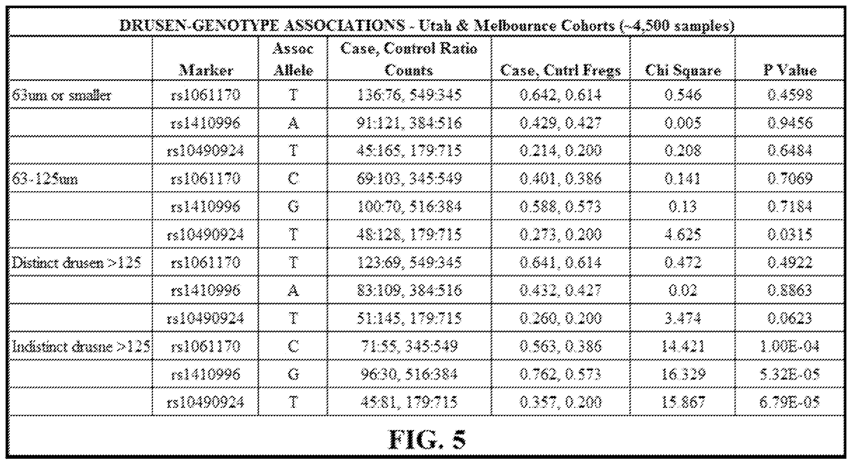

Figure 5 shows the association of various sizes of drusen with the AMD loci on chromosomes 1 and 10 in the combined cohort.

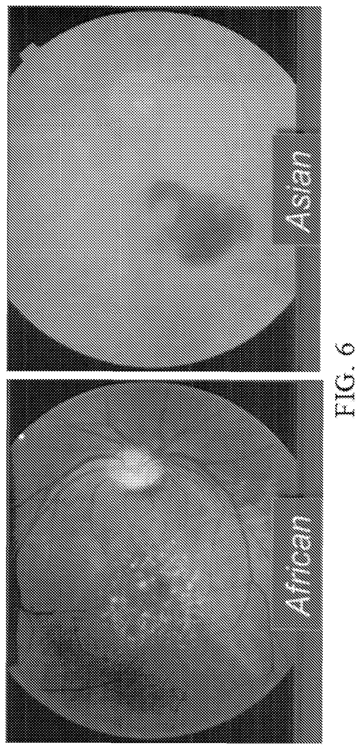

Figure 6 shows photos of eyes taken from AMD patients in different ethnic groups, indicating that AMD in Africans is associated with macular drusen, while AMD in Asians is primarily neovascular.
Figure 7 shows the association of geographic atrophy and choroidal neovascularization with chromosome 1 and chromosome 10 risk variants and haplotypes.

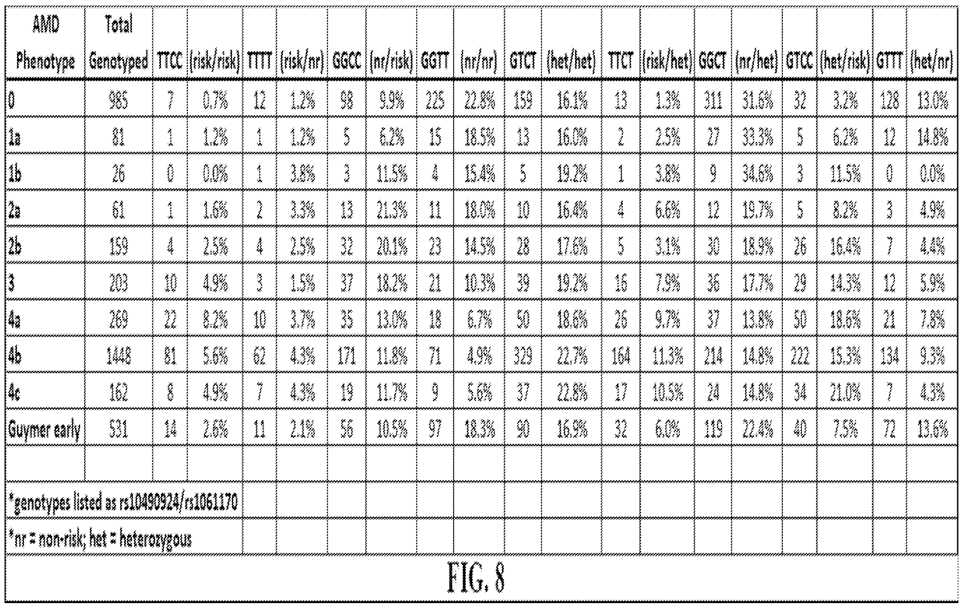

Figure 8 shows the percentage of each AMD eye phenotype that can be attributed to different chromosome 1/chromosome 10 genotypes.

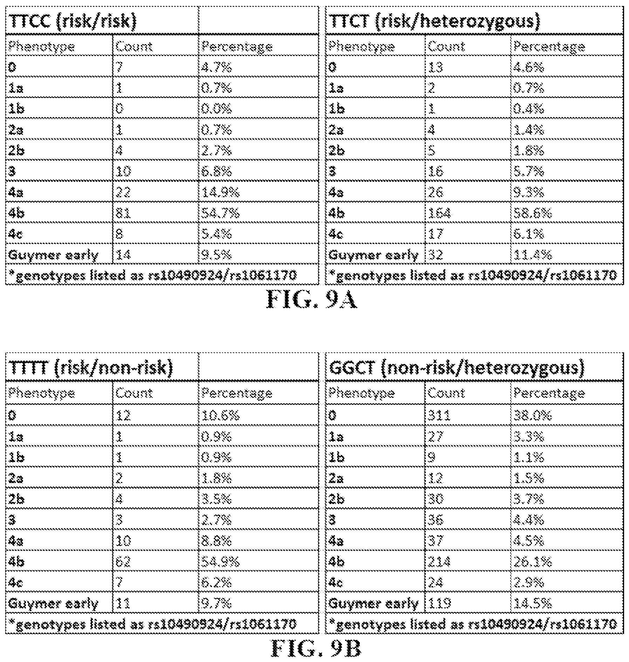

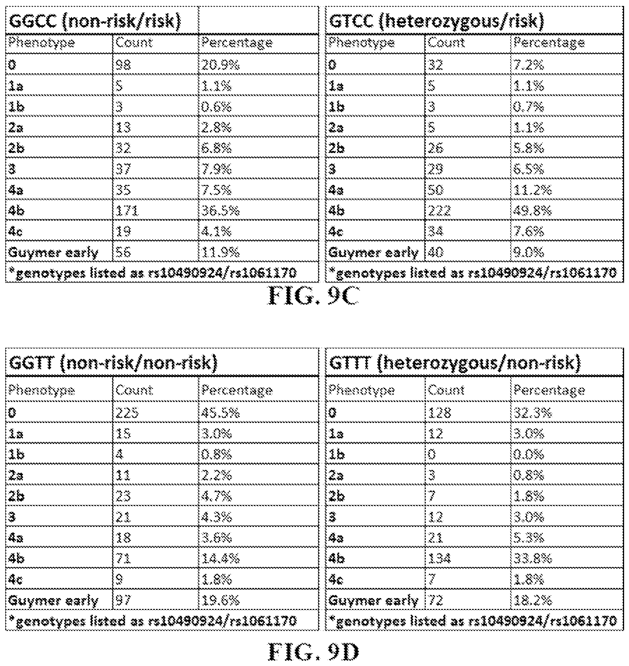

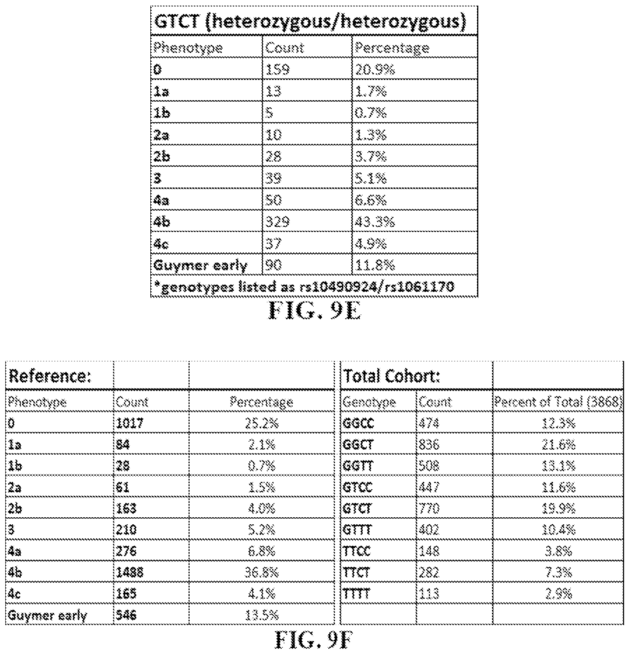

Figure 9 shows the percentage of each genotype combination that have certain AMD eye phenotypes.

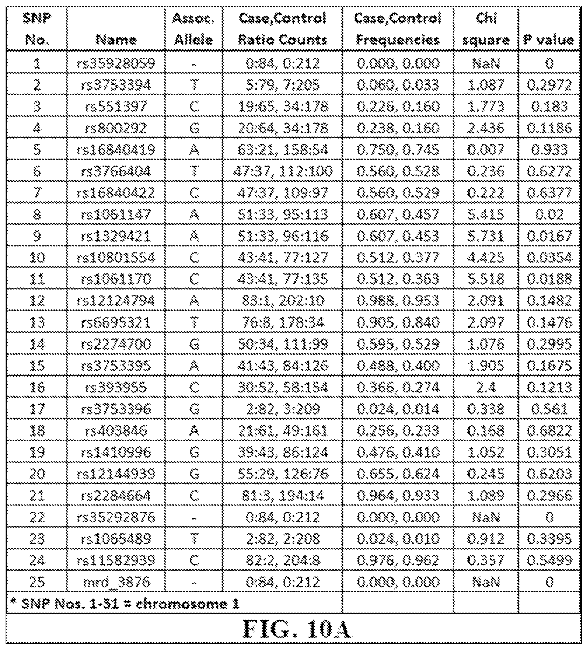

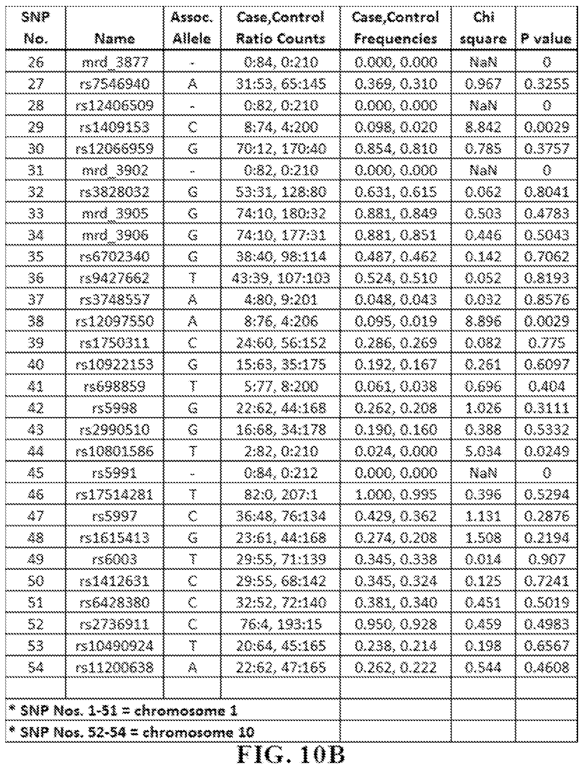

Figure 10 shows that chromosome 1 has an association with AMD in the Ghanaian cohort, while chromosome 10 does not.
Figure 11 shows the sequence of chromosome 1 haplotypes H1 through H5.

Additional advantages of the invention will be set forth in part in the description which follows, and in part will be obvious from the description, or can be learned by practice of the invention. The advantages of the invention will be realized and attained by means of the elements and combinations particularly pointed out in the appended claims.

### DESCRIPTION

The present invention as defined by the appended claims can be understood more readily by reference to the following detailed description of the invention and the Examples included therein.

### Definitions

As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values described herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10" as well as "greater than or equal to 10" is also disclosed. It is also understood that throughout the application, data are provided in a number of different formats, and that these data, represent endpoints, starting points, and ranges for any combination of the data points. For example, if a particular data point "10" and a particular data point 15 are disclosed, it is understood that greater than, greater than or equal to, less than, less than or equal to, and equal to 10 and 15 are considered disclosed as well as between 10 and 15. It is also understood that each unit between two particular units is also disclosed. For example, if 10 and 15 are disclosed, then 11, 12, 13, and 14 are also disclosed.

"Optional" or "optionally" means that the subsequently described event or circumstance may or may not occur and that the description includes instances where said event or circumstance occurs and instances where it does not.

The word "or" as used herein means any one member of a particular list and also includes any combination of members of that list.

"Haplotype" as used herein refers to a DNA sequence or a combination of DNA sequences present at various loci on a chromosome that are transmitted together; a haplotype may be one locus, several loci, or an entire chromosome depending on the number of recombination events that have occurred between a given set of loci.

"Risk Haplotype," "Risk SNP," or "Risk Diplotype" as used herein refers to a haplotype of a subject wherein the presence of the haplotype indicates the subject's increased risk of developing AMD when compared to a subject without said "Risk Haplotype," "Risk SNP," or "Risk Diplotype". A "Risk Haplotype," "Risk SNP," or "Risk Diplotype" also refers to a haplotype, SNP, or diplotype that occurs more often in subjects with AMD (cases) than in subjects without AMD (controls) in a given cohort. In other words, the case versus control percentage is different in cases than in controls as illustrated in the tables herein (See FIGS.). Although not required, the difference between the number of subjects with AMD (case) and the number of subjects without AMD (control) can be statistically significant.

"Protective Haplotype," "Protective SNP," or "Protective Diplotype" as used herein refers to a haplotype, SNP, or diplotype of a subject wherein the presence of the haplotype, SNP, or diplotype indicates the subject's decreased risk of developing AMD when compared to a subject without said protective haplotype. A "Protective Haplotype," "Protective SNP," or "Protective Diplotype" also refers to a haplotype, SNP, or diplotype that occurs less often in subjects with AMD (cases) than in subjects without AMD (controls), in other words, the case versus control percentage is lower in cases than in controls as illustrated in the tables herein (See FIGS.). Although not required, the difference between the number of subjects with AMD (case) and the number of subjects without AMD (control) can be statistically significant.

"Neutral Haplotype," "Neutral SNP," or "Neutral Diplotype" as used herein refers to a haplotype, SNP, or diplotype of a subject wherein the presence of the haplotype, SNP, or diplotype does not indicate the subject's increased or decreased risk of developing AMD. A "Neutral Haplotype," "Neutral SNP," or "Neutral Diplotype" also refers to a haplotype, SNP, or diplotype that occurs about equally in subjects with AMD (cases) and in subjects without AMD (controls), in other words, the case versus control percentage is about equal in cases and controls as illustrated in the tables herein (See FIGS.). Although not required, the difference between the number of subjects with AMD (case) and the number of subjects without AMD (control) can be non-statistically significant.

Haplotypes as described herein can be referred to by the generic nomenclature: "Hx_Y_Z", wherein "Hx" indicates the numbering of the specific haplotype as determined by the frequency of the specific haplotype in a given population or cohort. For example, H2 indicates a specific haplotype that occurs with the second highest frequency in the given data set, population or cohort. "Y", in the context of this nomenclature, specifies the SNP panel from which the overall haplotype was determined. "Z", in the context of this nomenclature, refers to the specific cohort used in ascertaining the specific haplotype.

"Major Haplotype" as used herein refers to a haplotype that occurs in greater than 1% of the population, data set or cohort being examined.

"Minor Haplotype" as used herein refers to a haplotype that occurs in 1% or less than 1% of the population, data set or cohort being examined.

"HapMap" as used herein refers to a haplotype map of the human genome that is a catalog of common genetic variants that occur in human beings. HapMap describes what these variants are, where they occur in DNA, how they distribute in haplotypes, and their frequencies among people within populations and among populations in different parts of the world. (The International HapMap Consortium. The International HapMap Project. Nature 426, 789-796 (2003)).

"HapMap Analysis" as used herein refers to an analysis that can be conducted using Haploview software (Barrett JC, Fry B, Maller J, Daly MJ. Haploview: analysis and visualization of LD and haplotype maps. Bioinformatics. 2005 Jan 15 [PubMed ID: 15297300]).

"Linkage Disequilibrium" as used herein refers to the non-random association between two or more alleles at two or more loci (not necessarily on the same chromosome) such that certain combinations of alleles are more likely to occur together on a chromosome than other combinations of alleles than would be expected from a random formation of haplotypes from alleles based on their frequencies.

### A. METHODS OF PREDICTING A SUBJECT'S RISK FOR HAVING OR DEVELOPING AMD

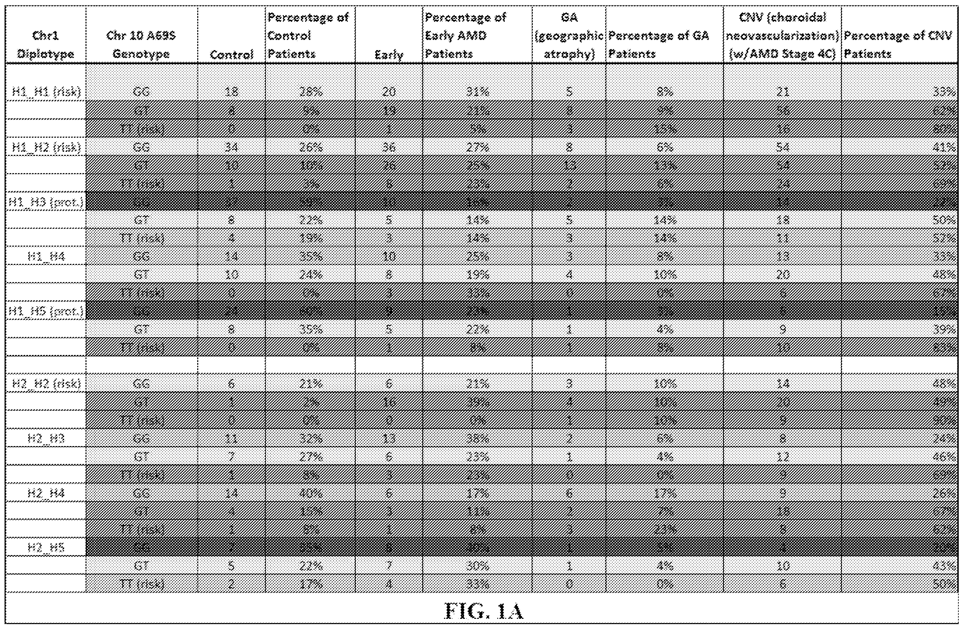

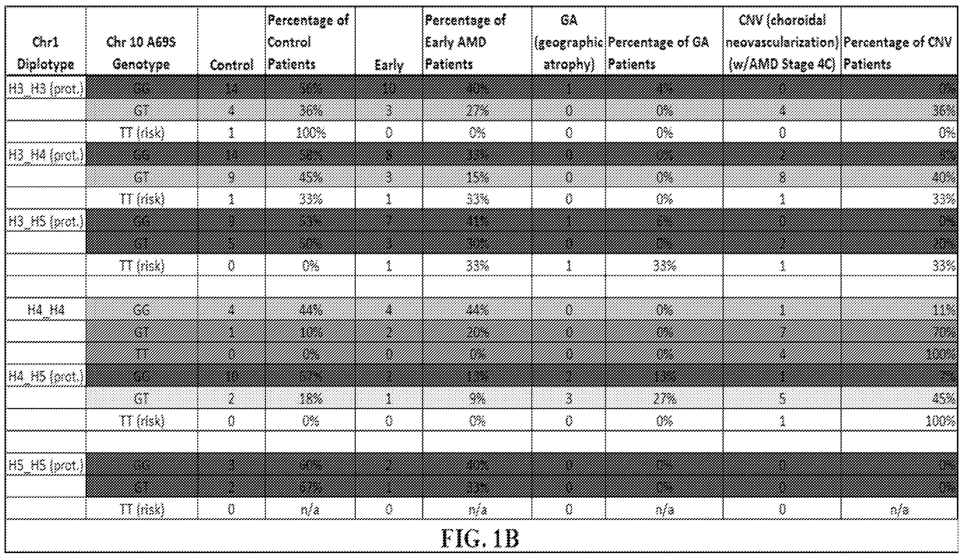

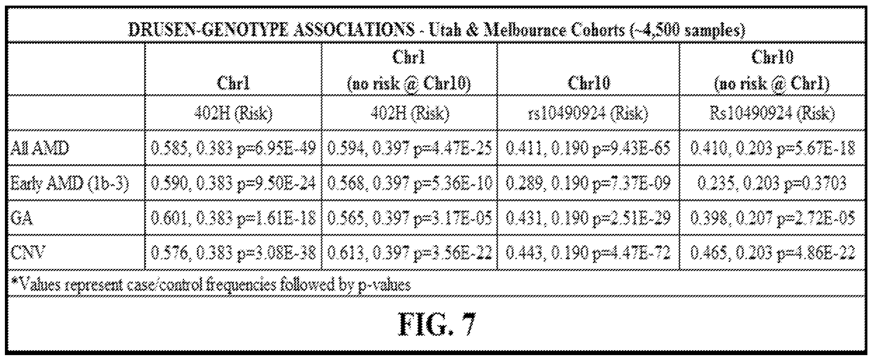

Described herein are methods and compositions relating to the discovery that there are two separate components that contribute to AMD, one from chromosome 1 (the complement component), and the other from chromosome 10 (the vascular component), and that, in certain cases, either component can drive the development and progression of disease independent of the other. This "two-disease" model of AMD has numerous important applications. For example, and not to be limiting, the methods and compositions described herein can be used to more accurately diagnose a subject with AMD, or to more accurately predict a subject's risk for having or developing AMD. In one aspect, as shown in Figure 7 herein, both the chromosome 1 locus and the chromosome 10 locus can affect the determination or diagnosis of disease in a subject. More specifically, Figure 7 shows that chromosome 10 risk factors do not drive the formation of drusen, and thus those subjects with only chromosome 10 risk, and no chromosome 1 risk, do not develop early disease. Accordingly, in those pure chromosome 10 patients, the formation of drusen does not need to be part of the AMD classification. In a further aspect, the methods described herein can be used to more accurately predict progression of AMD in a subject. In a further aspect, by utilizing the methods described herein, one can identify patients at risk for developing AMD that would have been missed if only assessing chromosome 1 factors or chromosome 10 factors alone. For example, as shown in Figure 1 herein, the chromosome 10 locus can be used to diagnose disease in a subject. More specifically, Figure 1 shows that individuals who carry one risk haplotype (HI for example) and one protective haplotype, in the absence of risk at chromosome 10, are strongly protected against the development of AMD, a situation that is not intuitive. Furthermore, in the presence of chromosome 10 risk, especially homozygous risk, and despite having the protective H1_H3 and H1_H5 chromosome 1 diplotypes, subjects having a chromosome 10 risk diplotype develop disease. Additionally, Figure 1 shows that, despite having a chromosome 10 risk diplotype, subjects having the protective H3_H3, H3_H4, and H3_H5 chromosome 1 diplotypes do not develop disease at as great a rate. Moreover, because it has been determined that occluded choriocapillaris lobules occur in the absence of both chromosome 1 and chromosome 10 risk factors, the methods described herein can be used to subdivide subjects with risk SNPs, haplotypes, or diplotypes on chromosome 1 into groups of subjects with or without occluded choriocapillaris lobules, and likewise to subdivide subjects with risk SNPs, haplotypes, or diplotypes on chromosome 10 into groups of subjects with or without occluded choriocapillaris lobules. Additionally, the methods and compositions described herein can be used to more accurately identify patients in need of particular types of treatments for AMD, for example complement-based treatments or vascular-based treatments. Thus, the methods described herein can enable a physician treating a subject for AMD to administer the appropriate AMD treatment to the subject by determining the subject's treatment based on the presence of or absence of certain SNPs, haplotypes, or diplotypes on chromosome 1 and chromosome 10 described herein. For example, and not to be limiting, administering the appropriate treatment to the subject could include not treating the subject with a complement-based therapy, or with an anti-VEGF therapy, if the subject has risk SNPs, haplotypes, or diplotypes on chromosome 10, but none on chromosome 1. For further example, administering the appropriate treatment could include not treating the subject with certain vitamin supplements, such as zinc supplements, if the subject has risk SNPs, haplotypes, or diplotypes on chromosome 10, but not on chromosome 1. Additionally, the methods and compositions described herein can be utilized by physicians and researchers conducting clinical trials for AMD when recruiting subjects for those clinical trials. Thus, in one aspect, the methods described herein can be used to identify appropriate subjects for AMD clinical trials, depending on whether the trial is related to a complement-based therapy or a vascular-based therapy. For example, and not to be limiting, using the methods described herein, a researcher can enroll a subject with risk SNPs, haplotypes, or diplotypes only on chromosome 1 into a clinical trial relating to a complement-based therapy, and can enroll a subject with risk SNPs, haplotypes, or diplotypes only on chromosome 10 into a clinical trial relating to a vascular-based therapy.

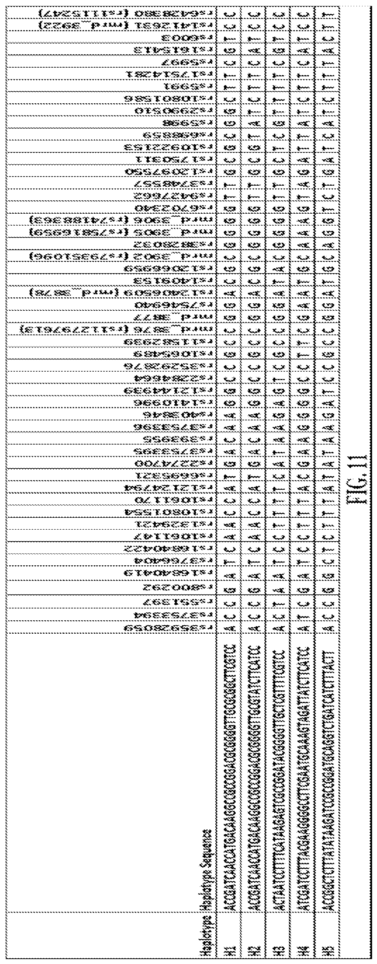

Described herein are methods for determining a subject's susceptibility to having or developing AMD comprising determining in the subject the identity of one or more chromosome 1 and chromosome 10 SNPs, haplotypes, or diplotypes described herein. In one aspect, the chromosome 1 SNP can be the rs 1061170 SNP in the complement factor H (CFH) gene, wherein the genotype can be CC, CT, or TT, and the chromosome 10 SNP can be the rs10490924 SNP in the HtrA serine protease 1 (HTRA1) gene, wherein the genotype can be GG, GT, or TT. In a further aspect, the chromosome 1 haplotype can be the haplotype H1 (SEQ ID NO. 1), H2 (SEQ ID NO. 2), H3 (SEQ ID NO. 3), H4 (SEQ ID NO. 4), or H5 (SEQ ID NO. 5), the sequences of which are set forth in FIG. 11, and the chromosome 10 haplotype can include the rs10490924 SNP in the HTRA1 gene, wherein the genotype can be GG, GT, or TT. In a further aspect, the chromosome 1 diplotype can be the diplotype H1_H1, H1_H2, H1_H3, H1_H4, H1_H5, H2_H2, H2_H3, H2_H4, H2_H5, H3_H3, H3_H4, H3_H5, H4_H4, H4_H5, or H5_H5, and the chromosome 10 diplotype can include the rs 10490924 SNP in HTRA1 gene, wherein the genotype can be GG, GT, or TT. The SNPs, haplotypes, or diplotypes can include a complement thereof. As used herein, "complement thereof' means a SNP, haplotype, or diplotype that is located on the complement DNA or RNA strand of any SNP, haplotype, or diplotype described herein. For example, and not to be limiting, the haplotype sequence "ATGC" can be converted to its complement thereof "TACG". Therefore, by way of example, if "ATGC" represents a haplotype indicative of a subject's susceptibility to having or developing AMD, its complement haplotype sequence, "TACG", also represents a haplotype indicative of a subject's susceptibility to having or developing AMD. As described herein, the presence of one or more of the SNPs, haplotypes, or diplotypes disclosed herein can indicate a subject's susceptibility for having or developing AMD.

In one aspect, determining the identity of certain chromosome 1 and chromosome 10 SNPs, haplotypes, or diplotypes in a subject can indicate that the subject has an increased risk of having or developing AMD. Such SNPs, haplotypes, and diplotypes can be risk SNPs, haplotypes, or diplotypes. For example, and not to be limiting, a chromosome 1 risk SNP can be the rs1061170 SNP in the CFH gene, wherein the genotype is CC, a chromosome 1 risk diplotype can be H1_H1, H1_H2, or H2_H2, a chromosome 10 risk SNP can be the rs10490924 SNP in HTRA1 gene, wherein the genotype is TT, and a chromosome 10 risk haplotype or diplotype can include the rs 10490924 SNP in HTRA1 gene, wherein the genotype is TT. Thus, described herein are methods for determining a subject's susceptibility to having or developing AMD comprising determining in the subject the identity of one or more chromosome 1 and chromosome 10 SNPs, haplotypes, or diplotypes described herein, wherein a risk SNP, haplotype, or diplotype is indicative of the subject's increased risk for having or developing AMD. In one aspect, also disclosed are methods of determining a subject's increased susceptibility to having or developing AMD, wherein the method further comprises administering a therapeutic composition to the subject when an increased risk is determined. In a further aspect, described herein are methods for determining a subject's AMD disease stage comprising determining in the subject the identity of one or more chromosome 1 and chromosome 10 SNPs, haplotypes, or diplotypes described herein.

In another aspect, determining the identity of certain chromosome 1 and chromosome 10 SNPs, haplotypes, or diplotypes in a subject can indicate that the subject has a decreased risk of having or developing AMD. Such SNPs, haplotypes, or diplotypes can be protective SNPs, haplotypes, or diplotypes. For example, and not to be limiting, a chromosome 1 protective SNP can be the rs1061170 SNP in the CFH gene, wherein the genotype is TT, a chromosome 1 protective diplotype can be H1_H3, H1_H5, H3_H3, H3_H4, H3_H5, H4_H5, or H5_H5, a chromosome 10 protective SNP can be the rs10490924 SNP in HTRA1 gene, wherein the genotype is GG, and a chromosome 10 protective haplotype or diplotype can include the rs10490924 SNP in HTRA1 gene, wherein the genotype is GG. Thus, described herein are methods for determining a subject's susceptibility to having or developing AMD comprising determining in the subject the identity of one or more chromosome 1 and chromosome 10 SNPs, haplotypes, or diplotypes described herein, wherein a protective SNP, haplotype, or diplotype is indicative of the subject's decreased risk for having or developing AMD.

In yet another aspect, determining the identity of certain chromosome 1 and chromosome 10 SNPs, haplotypes, or diplotypes in a subject can indicate that the subject does not have an increased risk or a decreased risk of having or developing AMD. Such SNPs, haplotypes, or diplotypes can be neutral SNPs, haplotypes, or diplotypes. For example, and not to be limiting, a chromosome 1 neutral SNP can be the rs1061170 SNP in the CFH gene, wherein the genotype is CT, a chromosome 1 neutral diplotype can be H1_H4, H2_H3, H2_H4, H2_H5, H3_H5, or H4_H4, a chromosome 10 neutral SNP, haplotype, or diplotype can be the rs10490924 SNP in HTRA1 gene, wherein the genotype is GT, and a chromosome 10 neutral haplotype, or diplotype can include the rs10490924 SNP in HTRA1 gene, wherein the genotype is GT. Thus, described herein are methods for determining a subject's susceptibility to having or developing AMD comprising determining in the subject the identity of one or more chromosome 1 and chromosome 10 SNPs, haplotypes, or diplotypes described herein, wherein a neutral SNP, haplotype, or diplotype does not indicate that the subject has an increased risk or a decreased risk for having or developing AMD.

As used herein, the term "subject" means an individual. In one aspect, a subject is a mammal such as a human. In a further aspect a subject can be a non-human primate. Non-human primates include marmosets, monkeys, chimpanzees, gorillas, orangutans, and gibbons, to name a few. The term "subject" also includes domesticated animals, such as cats, dogs, etc., livestock (for example, cattle (cows), horses, pigs, sheep, goats, etc.), laboratory animals (for example, ferret, chinchilla, mouse, rabbit, rat, gerbil, guinea pig, etc.) and avian species (for example, chickens, turkeys, ducks, pheasants, pigeons, doves, parrots, cockatoos, geese, etc.). Subjects can also include, but are not limited to fish (for example, zebrafish, goldfish, tilapia, salmon, and trout), amphibians and reptiles. As used herein, a "subject" is the same as a "patient," and the terms can be used interchangeably.

A haplotype can refer to a set of polymorphisms in a single gene, an intergenic sequence, or in larger sequences including both gene and intergenic sequences, e.g., a collection of genes, or of genes and intergenic sequences. For example, a haplotype can refer to a set of polymorphisms in either chromosome 1 genes or chromosome 10 genes.

As used herein the term "haplotype" can also refer to a set of single nucleotide polymorphisms (SNPs) found to be statistically associated with each other on a single chromosome. A haplotype can also refer to a combination of polymorphisms (e.g., SNPs) and other genetic markers (e.g., an insertion or a deletion) found to be statistically associated with each other on a single chromosome, for example, and not to be limiting, chromosome 1 or chromosome 10.

Human autosomal chromosomes normally come in pairs, and the combination in one individual of these two haplotypes is referred to herein as a "Diplotype". As such, "based on" when used in the context of a diplotype "based on" a haplotype, can refer to identifying the combination in one individual of these two haplotypes. Any haplotype or haplotypes described herein can be used to create a diplotype. For example, a diplotype can comprise a protective and a risk haplotype, two protective haplotypes, two risk haplotypes, a neutral and a risk haplotype, a neutral and a protective haplotype, or two neutral haplotypes. In an aspect, one haplotype may be dominant or one haplotype may be recessive.

The term "polymorphism" refers to the occurrence of one or more genetically determined alternative sequences or alleles in a population. A "polymorphic site" is the locus at which sequence divergence occurs. Polymorphic sites have at least one allele. A diallelic polymorphism has two alleles. A triallelic polymorphism has three alleles. Diploid organisms may be homozygous or heterozygous for allelic forms. A polymorphic site can be as small as one base pair. Examples of polymorphic sites include: restriction fragment length polymorphisms (RFLPs), variable number of tandem repeats (VNTRs), hypervariable regions, minisatellites, dinucleotide repeats, trinucleotide repeats, tetranucleotide repeats, and simple sequence repeats. As used herein, reference to a "polymorphism" can encompass a set of polymorphisms (i.e., a haplotype).

A "single nucleotide polymorphism (SNP)" can occur at a polymorphic site occupied by a single nucleotide, which is the site of variation between allelic sequences. The site can be preceded by and followed by highly conserved sequences of the allele. A SNP can arise due to substitution of one nucleotide for another at the polymorphic site. Replacement of one purine by another purine or one pyrimidine by another pyrimidine is called a transition. Replacement of a purine by a pyrimidine or vice versa is called a transversion. A synonymous SNP refers to a substitution of one nucleotide for another in the coding region that does not change the amino acid sequence of the encoded polypeptide. A non-synonymous SNP refers to a substitution of one nucleotide for another in the coding region that changes the amino acid sequence of the encoded polypeptide. A SNP may also arise from a deletion or an insertion of a nucleotide or nucleotides relative to a reference allele.

A "set" of polymorphisms means one or more polymorphism, e.g., at least 1, at least 2, at least 3, at least 4, at least 5, at least 6, or more than 6 polymorphisms.

As used herein, a "nucleic acid," "polynucleotide," or "oligonucleotide" can be a polymeric form of nucleotides of any length, can be DNA or RNA, and can be single- or double-stranded. Nucleic acids can include promoters or other regulatory sequences. Oligonucleotides can be prepared by synthetic means. Nucleic acids include segments of DNA, or their complements spanning or flanking any one of the polymorphic sites described herein. The segments can be between 5 and 100 contiguous bases and can range from a lower limit of 5, 10, 15, 20, or 25 nucleotides to an upper limit of 10, 15, 20, 25, 30, 50, or 100 nucleotides (where the upper limit is greater than the lower limit). Nucleic acids between 5-10, 5-20, 10-20, 12-30, 15-30, 10-50, 20-50, or 20-100 bases are common. The polymorphic site can occur within any position of the segment. A reference to the sequence of one strand of a double-stranded nucleic acid defines the complementary sequence and except where otherwise clear from context, a reference to one strand of a nucleic acid also refers to its complement.

"Nucleotide" as described herein refers to molecules that, when joined, make up the individual structural units of the nucleic acids RNA and DNA. A nucleotide is composed of a nucleobase (nitrogenous base), a five-carbon sugar (either ribose or 2-deoxyribose), and one phosphate group. "Nucleic acids" are polymeric macromolecules made from nucleotide monomers. In DNA, the purine bases are adenine (A) and guanine (G), while the pyrimidines are thymine (T) and cytosine (C). RNA uses uracil (U) in place of thymine (T).

Exemplary polymorphic sites on chromosome 1 and chromosome 10 are described herein as examples and are not intended to be limiting. These polymorphic sites, SNPs, haplotypes, or diplotypes can also be used in carrying out the methods described herein. Moreover, it will be appreciated that these polymorphisms are useful for linkage and association studies, genotyping clinical populations, correlation of genotype information to phenotype information, loss of heterozygosity analysis, identification of the source of a cell sample, and can also be useful to target potential therapeutics to cells.

It will be appreciated that additional polymorphic sites, which are not explicitly described herein, may further refine this analysis. A SNP analysis using non-synonymous polymorphisms can be useful to identify variant polypeptides. Other SNPs associated with risk may encode a protein with the same sequence as a protein encoded by a neutral or protective SNP but contain an allele in a promoter or intron, for example, changes the level or site of gene expression. It will also be appreciated that a polymorphism described herein may be linked to a variation in a neighboring gene. The variation in the neighboring gene may result in a change in expression or form of an encoded protein and have detrimental or protective effects in the carrier.

Also described herein are methods of screening for polymorphic sites in other genes that are in linkage disequilibrium (LD) with a risk, protective, or otherwise informative SNP or genetic marker described herein, including but not limited to the polymorphic sites in the genes described herein. These methods can involve identifying a polymorphic site in a gene that is in linkage disequilibrium with a polymorphic site in the genes described herein, wherein the polymorphic form of the polymorphic site in the gene described herein is associated with AMD, (e.g., increased or decreased risk), and determining SNPs, haplotypes, and diplotypes in a population of individuals to indicate whether the linked polymorphic site has a polymorphic form in linkage disequilibrium with the polymorphic form of the gene that correlates with the AMD phenotype.

As used herein, "linkage disequilibrium" is the non-random association of alleles at two or more loci, not necessarily on the same chromosome. It is not the same as linkage, which describes the association of two or more loci on a chromosome with limited recombination between them. Linkage disequilibrium describes a situation in which some combinations of alleles or genetic markers occur more or less frequently in a population than would be expected from a random formation of haplotypes from alleles based on their frequencies. Non-random associations between polymorphisms at different loci are measured by the degree of linkage disequilibrium (LD). The level of linkage disequilibrium can be influenced by a number of factors including genetic linkage, the rate of recombination, the rate of mutation, random drift, non-random mating, and population structure. "Linkage disequilibrium" or "allelic association" thus means the non-random association of a particular allele or genetic marker with another specific allele or genetic marker more frequently than expected by chance for any particular allele frequency in the population. A marker in linkage disequilibrium with an informative marker, such as one of the SNPs, haplotypes, or diplotypes described herein can be useful in detecting susceptibility to complement-mediated disease. A SNP that is in linkage disequilibrium with a risk, protective, or otherwise informative SNP, haplotype, diplotype, or genetic marker described herein can be referred to as a "proxy" or "surrogate" SNP. A proxy SNP may be in at least 50%, 60%, or 70% in linkage disequilibrium with risk, protective, or otherwise informative SNP or genetic marker described herein, and in one aspect is at least about 80%, 90%, and in another aspect 91%, 92%, 93%, 94%, 95%, 96%, 97%, 98%, 99%, or about 100% in LD with a risk, protective, or otherwise informative SNP, haplotype, diplotype, or genetic marker described herein.

Publicly available databases such as the HapMap database (http://ftp.hapmap.org/ld_data/latest/) and Haploview (Barrett, J. C. et al., Bioinformatics 21, 263 (2005)) may be used to calculate linkage disequilibrium between two SNPs. The frequency of identified alleles in disease versus control populations can be determined using the methods described herein. Statistical analyses can be employed to determine the significance of a non-random association between the two SNPs (e.g., Hardy-Weinberg Equilibrium, Genotype likelihood ratio (genotype p value), Chi Square analysis, Fishers Exact test). A statistically significant non-random association between the two SNPs indicates that they are in linkage disequilibrium and that one SNP can serve as a proxy for the second SNP.

In another aspect, polymorphisms in the genes described herein can be used to establish physical linkage between a genetic locus associated with a trait of interest and polymorphic markers that are not associated with the trait, but are in physical proximity with the genetic locus responsible for the trait and co-segregate with it. Mapping a genetic locus associated with a trait of interest facilitates cloning the gene(s) responsible for the trait following procedures that are well-known in the art.

As used herein, "physical linkage" describes the tendency of genes, alleles, loci or genetic markers to be inherited together as a result of their location on the same chromosome. Linkage can be measured by percent recombination between the two genes, alleles, loci or genetic markers. Typically, loci occurring within a 50 centimorgan (cM) distance of each other are linked. Linked markers may occur within the same gene or gene cluster.

As used herein, the term "variant" can also refer to a nucleotide sequence in which the sequence differs from the sequence most prevalent in a population, for example by one nucleotide, in the case of the SNPs described herein. For example, some variations or substitutions in the nucleotide sequence of the genes described herein alter a codon so that a different amino acid is encoded resulting in a variant polypeptide. The term "variant," can also refer to a polypeptide in which the sequence differs from the sequence most prevalent in a population at a position that does not change the amino acid sequence of the encoded polypeptide (i.e., a conserved change). Variant polypeptides can be encoded by a risk haplotype, encoded by a protective haplotype, or can be encoded by a neutral haplotype. Variant polypeptides can be associated with risk, associated with protection, or can be neutral.

The methods and materials described herein can be used to determine whether the nucleic acid of a subject (e.g., human) contains a polymorphism, such as a single nucleotide polymorphism (SNP). For example, methods and materials provided herein can be used to determine whether a subject has a variant SNP. Any method known in the art can be used to detect a polymorphism in the genes described herein. For example, polymorphisms can be detected by sequencing exons, introns, or untranslated sequences, denaturing high performance liquid chromatography (DHPLC), allele-specific hybridization, allele-specific restriction digests, mutation specific polymerase chain reactions, single-stranded conformational polymorphism detection, and combinations of such methods.

In one aspect, polymorphisms can be detected in a target nucleic acid isolated from a subject. Typically genomic DNA is analyzed. For assay of genomic DNA, virtually any biological sample containing genomic DNA or RNA, e.g., nucleated cells, is suitable. Suitable samples include, but are not limited to, saliva, cheek scrapings, biopsies of retina, kidney or liver or other organs or tissues; skin biopsies; amniotic fluid or CNS samples; and the like. In one aspect RNA or cDNA can be assayed. In one aspect, the assay can detect variant proteins encoded by one or more genes described herein. Methods for purification or partial purification of nucleic acids or proteins from patient samples for use in diagnostic or other assays are known in the art.

By "isolated nucleic acid" or "purified nucleic acid" is meant DNA that is free of the genes that, in the naturally-occurring genome of the organism flank the gene. The term therefore includes, for example, a recombinant DNA which is incorporated into a vector, such as an autonomously replicating plasmid or virus; or incorporated into the genomic DNA of a prokaryote or eukaryote (e.g., a transgene); or which exists as a separate molecule (for example, a cDNA or a genomic or cDNA fragment produced by PCR, restriction endonuclease digestion, or chemical or in vitro synthesis). It also includes a recombinant DNA which is part of a hybrid gene encoding additional polypeptide sequence. The term "isolated nucleic acid" also refers to RNA, e.g., an mRNA molecule that is encoded by an isolated DNA molecule, or that is chemically synthesized, or that is separated or substantially free from at least some cellular components, for example, other types of RNA molecules or polypeptide molecules.

The identity of bases occupying the polymorphic sites in the genes described herein can be determined in a subject, e.g., in a patient being analyzed, using any of several methods known in the art. For example, and not to be limiting use of allele-specific probes, use of allele-specific primers, direct sequence analysis, denaturing gradient gel electrophoresis (DGGE) analysis, single-strand conformation polymorphism (SSCP) analysis, and denaturing high performance liquid chromatography (DHPLC) analysis. Other well-known methods to detect polymorphisms in DNA include use of: Molecular Beacons technology (see, e.g., Piatek et al., 1998; Nat. Biotechnol. 16:359-63; Tyagi, and Kramer, 1996, Nat. Biotechnology 14:303-308; and Tyagi, et al., 1998, Nat. Biotechnol. 16:49-53), Invader technology (see, e.g., Neri et al., 2000, Advances in Nucleic Acid and Protein Analysis 3826:117-125 and U.S. Pat. No. 6,706,471), nucleic acid sequence based amplification (Nasba) (Compton, 1991), Scorpion technology (Thelwell et al., 2000, Nuc. Acids Res, 28:3752-3761 and Solinas et al., 2001, "Duplex Scorpion primers in SNP analysis and FRET applications" Nuc. Acids Res, 29:20.), restriction fragment length polymorphism (RFLP) analysis, and the like. Additional methods will be apparent to one of skill in the art.

The term "primer" refers to a single-stranded oligonucleotide capable of acting as a point of initiation of template-directed DNA synthesis under appropriate conditions, in an appropriate buffer and at a suitable temperature. The appropriate length of a primer depends on the intended use of the primer but typically ranges from 15 to 30 nucleotides. A primer sequence need not be exactly complementary to a template but must be sufficiently complementary to hybridize with a template. The term "primer site" refers to the area of the target DNA to which a primer hybridizes. The term "primer pair" means a set of primers including a 5' upstream primer, which hybridizes to the 5' end of the DNA sequence to be amplified and a 3' downstream primer, which hybridizes to the complement of the 3' end of the sequence to be amplified.

Exemplary hybridization conditions for short probes and primers is about 5 to 12 degrees C, below the calculated Tm. Formulas for calculating Tm are known and include: Tm = 4°C x (number of G's and C's in the primer) + 2°C x (number of A's and T's in the primer) for oligos <14 bases and assumes a reaction is carried out in the presence of 50 mM monovalent cations. For longer oligos, the following formula can be used: Tm = 64.9°C + 41°C x (number of G's and C's in the primer-16.4)/N, where N is the length of the primer. Another commonly used formula takes into account the salt concentration of the reaction (Rychlik, supra, Sambrook, supra, Mueller, supra.): Tm = 81.5°C + 16.6°C x (log 10[Na+]+[K+]) + 0.41°C x (% GC) - 675/N, where N is the number of nucleotides in the oligo. The aforementioned formulas provide a starting point for certain applications; however, the design of particular probes and primers may take into account additional or different factors. Methods for design of probes and primers for use in the present methods are well known in the art.

The design and use of allele-specific probes for analyzing polymorphisms are described by e.g., Saiki et al., 1986; Dattagupta, EP 235,726, Saiki, WO 89/11548. Briefly, allele-specific probes are designed to hybridize to a segment of target DNA from one individual but not to the corresponding segment from another individual, if the two segments represent different polymorphic forms. Hybridization conditions are chosen that are sufficiently stringent so that a given probe essentially hybridizes to only one of two alleles. Typically, allele-specific probes can be designed to hybridize to a segment of target DNA such that the polymorphic site aligns with a central position of the probe.

As used herein, "probes" are nucleic acids capable of binding in a base-specific manner to a complementary strand of nucleic acid. Such probes include nucleic acids and peptide nucleic acids (Nielsen et al., 1991). Hybridization may be performed under stringent conditions which are known in the art. For example, see Berger and Kimmel (1987) Methods In Enzymology, Vol. 152: Guide To Molecular Cloning Techniques, San Diego: Academic Press, Inc.; Sambrook et al. (1989) Molecular Cloning: A Laboratory Manual, 2nd Ed., Vols. 1-3, Cold Spring Harbor Laboratory; Sambook (2001) 3rd Edition; Rychlik, W. and Rhoads, R. E., 1989, Nucl. Acids Res. 17, 8543; Mueller, P. R. et al. (1993) In: Current Protocols in Molecular Biology 15.5, Greene Publishing Associates, Inc. and John Wiley and Sons, New York; and Anderson and Young, Quantitative Filter Hybridization in Nucleic Acid Hybridization (1985)). As used herein, the term "probe" includes primers. Probes and primers are sometimes referred to as "oligonucleotides."

Allele-specific probes can be used in pairs, one member of a pair designed to hybridize to the reference allele of a target sequence and the other member designed to hybridize to the variant allele. Several pairs of probes can be immobilized on the same support for simultaneous analysis of multiple polymorphisms within the same target gene sequence.

The design and use of allele-specific primers for analyzing polymorphisms are described by, e.g., WO 93/22456 and Gibbs, 1989. Briefly, allele-specific primers are designed to hybridize to a site on target DNA overlapping a polymorphism and to prime DNA amplification according to standard PCR protocols only when the primer exhibits perfect complementarity to the particular allelic form. A single-base mismatch prevents DNA amplification and no detectable PCR product is formed. The method works best when the polymorphic site is at the extreme 3'-end of the primer, because this position is most destabilizing to elongation from the primer. Any of the primers and probes described herein can be used in the methods described herein.

In some embodiments, genomic DNA can be used to detect polymorphisms in the genes described herein. Genomic DNA can be extracted from a sample, such as a peripheral blood sample or a tissue sample. Standard methods can be used to extract genomic DNA from a sample, such as phenol extraction. In some cases, genomic DNA can be extracted using a commercially available kit (e.g., from Qiagen, Chatsworth, Calif.; Promega, Madison, Wis.; or Gentra Systems, Minneapolis, Minn.).

Other methods for detecting polymorphisms can involve amplifying a nucleic acid from a sample obtained from a subject (e.g., amplifying the segments of nucleic acids from the genes described herein using specific primers) and analyzing the amplified nucleic acids. This can be accomplished by standard polymerase chain reaction (PCR, qPCT, & RT-PCR) protocols or other methods known in the art. The amplifying can result in the generation of allele-specific oligonucleotides, which span the single nucleotide polymorphic sites in the genes described herein. The specific primer sequences allele-specific oligonucleotides can be derived from the coding (exons) or non-coding (promoter, 5' untranslated, introns or 3' untranslated) regions of the genes described herein. In one aspect Genomic DNA from all subjects can be isolated from peripheral blood leukocytes with QIAamp DNA Blood Maxi kits (Qiagen, Valencia, CA). DNA samples can be screened for SNPs in the genes described herein. Genotyping can be performed byTaqMan assays (Applied Biosystems, Foster City, California) using 10 ng of template DNA in a 5uL reaction. The thermal cycling conditions in the 384-well thermocycler (PTC-225, MJ Research) can consist of an initial hold at 95°C for 10 minutes, followed by 40 cycles of a 15-second 95°C denaturation step and a 1-minute 60°C annealing and extension step. Plates can be read in the 7900HT Fast Real-Time PCR System (Applied Biosystems).

Amplification products generated using PCR can be analyzed by the use of denaturing gradient gel electrophoresis (DGGE). Different alleles can be identified based on sequence-dependent melting properties and electrophoretic migration in solution. See Erlich, ed., PCR Technology, Principles and Applications for DNA Amplification, Chapter 7 (W.H. Freeman and Co, New York, 1992).

Alleles of target sequences can be differentiated using single-strand conformation polymorphism (SSCP) analysis. Different alleles can be identified based on sequence- and structure-dependent electrophoretic migration of single stranded PCR products (Orita et al., 1989). Amplified PCR products can be generated according to standard protocols and heated or otherwise denatured to form single stranded products, which may refold or form secondary structures that are partially dependent on base sequence.

Alleles of target sequences can be differentiated using denaturing high performance liquid chromatography (DHPLC) analysis. Different alleles can be identified based on base differences by alteration in chromatographic migration of single stranded PCR products (Frueh and Noyer-Weidner, 2003). Amplified PCR products can be generated according to standard protocols and heated or otherwise denatured to form single stranded products, which may refold or form secondary structures that are partially dependent on the base sequence.

Direct sequence analysis of polymorphisms can be accomplished using DNA sequencing procedures that are well-known in the art. See Sambrook et al., Molecular Cloning, A Laboratory Manual (2nd Ed., CSHP, New York 1989) and Zyskind et al., Recombinant DNA Laboratory Manual (Acad. Press, 1988). In one aspect, the DNA sequencing procedure can be DNA sequencing by synthesis. In a further aspect, the DNA sequencing procedure can be high-throughput or Next Generartion Sequencing (NGS). Unlike PCR, microarrays, high-resolution melting, and mass spectrometry, which all indirectly infer sequence content, NGS directly ascertains the identity of each base and the order in which they fall within a gene. The newest platforms on the market have the capacity to cover an exonic region 10,000 times over, meaning the content of each base position in the sequence is measured thousands of different times. This high level of coverage ensures that the consensus sequence is extremely accurate and enables the detection of rare variants within a heterogeneous sample. For example, in a sample extracted from FFPE tissue, relevant mutations are only present at a frequency of 1% with the wild-type allele comprising the remainder. When this sample is sequenced at 10,000X coverage, then even the rare allele, comprising only 1% of the sample, is uniquely measured 100 times over. Thus, NGS can provide reliably accurate results with very high sensitivity, making it ideal for clinical diagnostic testing of FFPEs and other mixed samples.

Examples of Next Generation Sequencing techniques include, but are not limited to Massively Parallel Signature Sequencing (MPSS), Polony sequencing, pyrosequencing, Reversible dye-terminator sequencing, SOLiD sequencing, Ion semiconductor sequencing, DNA nanoball sequencing, Helioscope single molecule sequencing, Single molecule real time (SMRT) sequencing, Single molecule real time (RNAP) sequencing, and Nanopore DNA sequencing.

A wide variety of other methods are known in the art for detecting polymorphisms in a biological sample. See, e.g., Ullman et al. "Methods for single nucleotide polymorphism detection" U.S. Pat. No. 6,632,606; Shi, 2002, "Technologies for individual genotyping: detection of genetic polymorphisms in drug targets and disease genes" Am J Pharmacogenomics 2:197-205; Kwok et al., 2003, "Detection of single nucleotide polymorphisms" Curr Issues Biol. 5:43-60).

In one aspect, the chromosome 1 and chromosome 10 SNPs, haplotypes, or diplotypes described herein can be determined from a sample obtained from a subject. In another aspect, a subject's chromosome 1 and chromosome 10 SNPs, haplotypes, or diplotypes can be determined by amplifying or sequencing a nucleic acid sample obtained from the subject. In yet a further aspect, the methods described herein can further comprise administering a therapeutic composition to the subject or treating the subject with an effective amount of a therapeutic composition. In still a further aspect, the methods described herein can comprise administering a therapeutic composition to a subject after diagnosing the subject with AMD based upon the presence of any of the chromosome 1 and chromosome 10 SNPs, haplotypes, or diplotypes described herein. In another aspect, the methods described herein can comprise administering a therapeutic composition to a subject after determining an appropriate AMD treatment for the subject, or after enrolling the subject into the appropriate AMD clinical trial, based upon the presence of any of the chromosome 1 and chromosome 10 SNPs, haplotypes, or diplotypes described herein.

As used herein, "treating" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. In various aspects, the term covers any treatment of a subject, including a mammal (e.g., a human), and includes: (i) preventing the disease from occurring in a subject that can be predisposed to the disease but has not yet been diagnosed as having it; (ii) inhibiting the disease, i.e., arresting its development; or (iii) relieving the disease, i.e., causing regression of the disease.

The terms "administering" and "administration" refer to any method of providing a pharmaceutical preparation to a subject. Such methods are well known to those skilled in the art and include, but are not limited to, oral administration, sublingual administration, trans-buccal mucosa administration, transdermal administration, administration by inhalation, nasal administration, topical administration, intravaginal administration, ophthalmic administration, intraaural administration, intracerebral administration, intrathecal administration, rectal administration, intraperitoneal administration, and parenteral administration, including injectable such as intravenous administration, intra-arterial administration, intramuscular administration, intradermal administration, and subcutaneous administration. Ophthalmic administration can include topical administration, subconjunctival administration, sub-Tenon's administration, epibulbar administration, retrobulbar administration, intra-orbital administration, and intraocular administration, which includes intra-vitreal administration. Administration can be continuous or intermittent. In various aspects, a preparation can be administered therapeutically; that is, administered to treat an existing disease or condition. In further various aspects, a preparation can be administered prophylactically; that is, administered for prevention of a disease or condition.

As used herein, the term "therapeutically effective amount" refers to an amount that is sufficient to achieve the desired result or to have an effect on an undesired condition.

In yet another aspect, the methods for determining a subject's susceptibility to having or developing AMD herein can further comprise examining the subject with an ophthalmological procedure.

Various ophthalmological procedures known to persons of ordinary skill in the art can be performed to examine the subject including, but not limited to, autofluorescent imaging techniques, infrared imaging techniques, optical coherence tomography (OCT), Stratus optical coherence tomography (Stratus OCT), Fourier-domain optical coherence tomography (Fd-OCT), two-photon-excited fluorescence (TPEF) imaging, adaptive optics scanning laser ophthalmoscopy (AOSLO), scanning laser ophthalmoscopy, near-infrared imaging combined with spectral domain optical coherence tomography (SD-OCT), color fundus photography, fundus autofluorescence imaging, red-free imaging, fluorescein angiography, indocyanin green angiography, multifocal electroretinography (ERG) recording, microperimetry, color Doppler optical coherence tomography (CDOCT), and visual field assessment. Additionally, the subject can be examined by using the Heidelberg Spectralis, the Zeiss Cirrus, the Topcon 3D OCT 2000, the Optivue RTVue SD-OCT, the Opko OCT SLO, the NIDEK F-10, or the Optopol SOCT Copernicus HR.

Also described herein are methods for predicting progression of AMD in a subject comprising determining in the subject the identity of one or more of the chromosome 1 and chromosome 10 SNPs, haplotypes, or diplotypes described herein. In one aspect, determining the identity of certain chromosome 1 and chromosome 10 SNPs, haplotypes, or diplotypes in a subject can indicate that the subject has an increased risk of having or developing late-stage age-related macular degeneration. Such SNPs, haplotypes, or diplotypes can be risk SNPs, haplotypes, or diplotypes. Thus, described herein are methods for determining a subject's susceptibility to having or developing late-stage age-related macular degeneration comprising determining in the subject the identity of one or more chromosome 1 and chromosome 10 SNPs, haplotypes, or diplotypes described herein, wherein a risk SNP, haplotype, or diplotype is indicative of the subject's increased risk for having or developing late-stage age-related macular degeneration.

In another aspect, determining the identity of certain chromosome 1 and chromosome 10 SNPs, haplotypes, or diplotypes in a subject can indicate that the subject has a decreased risk of having or developing late-stage age-related macular degeneration. Such SNPs, haplotypes, or diplotypes can be protective SNPs, haplotypes, or diplotypes. Thus, described herein are methods for determining a subject's susceptibility to having or developing late-stage age-related macular degeneration comprising determining in the subject the identity of one or more chromosome 1 and chromosome 10 SNPs, haplotypes, or diplotypes described herein, wherein a protective SNP, haplotype, or diplotype is indicative of the subject's decreased risk for having or developing late-stage age-related macular degeneration.

In yet another aspect, determining the identity of certain chromosome 1 and chromosome 10 SNPs, haplotypes, or diplotypes in a subject can indicate that the subject does not have an increased risk or a decreased risk of having or developing late-stage age-related macular degeneration. Such SNPs, haplotypes, or diplotypes can be neutral SNPs, haplotypes, or diplotypes. Thus, described herein are methods for determining a subject's susceptibility to having or developing late-stage age-related macular degeneration comprising determining in the subject the identity of one or more chromosome 1 and chromosome 10 SNPs, haplotypes, or diplotypes described herein, wherein a neutral SNP, haplotype, or diplotype does not indicate that the subject has an increased risk or a decreased risk for having or developing late-stage age-related macular degeneration.

### B. METHODS OF IDENTIFYING A SUBJECT IN NEED OF TREATMENT FOR AGE-RELATED MACULAR DEGENERATION

Described herein are methods of identifying a subject in need of treatment for AMD comprising determining in the subject the identity of one or more chromosome 1 and chromosome 10 SNPs, haplotypes, or diplotypes described herein. In one aspect, the chromosome 1 SNP can be the rs1061170 SNP in the CFH gene, wherein the genotype can be CC, CT, or TT, and the chromosome 10 SNP can be the rs10490924 SNP in the HTRA1 gene, wherein the genotype can be GG, GT, or TT. In a further aspect, the chromosome 1 haplotype can be the haplotype H1 (SEQ ID NO. 1), H2 (SEQ ID NO. 2), H3 (SEQ ID NO. 3), H4 (SEQ ID NO. 4), or H5 (SEQ ID NO. 5), the sequences of which are set forth in FIG. 11, and the chromosome 10 haplotype can include the rs10490924 SNP in the HTRA1 gene, wherein the genotype can be GG, GT, or TT. In a further aspect, the chromosome 1 diplotype can be the diplotype H1_H1, H1_H2, H1_H3, H1_H4, H1_H5, H2_H2, H2_H3, H2_H4, H2_H5, H3_H3, H3_H4, H3_H5, H4_H4, H4_H5, or H5_H5, and the chromosome 10 diplotype can include the rs10490924 SNP in HTRA1, wherein the genotype can be GG, GT, or TT. As described herein, the presence of one or more of the chromosome 1 and chromosome 10 SNPs, haplotypes, or diplotypes disclosed herein can indicate whether a subject is in need of treatment for AMD. As used herein, "treatment" can also mean prophylactic, or preventative treatment.

In one aspect, determining the identity of certain chromosome 1 and chromosome 10 SNPs, haplotypes, or diplotypes in a subject can indicate that the subject is in need of treatment for AMD. Such SNPs, haplotypes, or diplotypes can be risk SNPs, haplotypes, or diplotypes. Thus, described herein are methods for identifying a subject in need of treatment for AMD comprising determining in the subject the identity of one or more chromosome 1 and chromosome 10 SNPs, haplotypes, or diplotypes described herein, wherein a risk SNP, haplotype, or diplotype can indicate that the subject is in need of treatment. In one aspect, the method of identifying a subject in need of treatment can further comprise administering a therapeutic composition to the subject identified as being in need of treatment. In another aspect, the methods described herein can be used to identify a subject in need of more frequent screening for AMD. For example, identifying a subject with a risk chromosome 1 and chromosome 10 SNP, haplotype, or diplotype can indicate that the subject needs to be more closely and frequently monitored for the development of AMD.

In another aspect, determining the identity of certain chromosome 1 and chromosome 10 SNPs, haplotypes, or diplotypes in a subject can indicate that the subject is not in need of treatment for AMD. Such SNPs, haplotypes, or diplotypes can be protective SNPs, haplotypes, or diplotypes. Thus, described herein are methods for identifying a subject in need of treatment for AMD comprising determining in the subject the identity of one or more chromosome 1 and chromosome 10 SNPs, haplotypes, or diplotypes described herein, wherein a protective SNP, haplotype, or diplotype can indicate that the subject is not in need of treatment for AMD.

In yet another aspect, determining the identity of certain chromosome 1 and chromosome 10 SNPs, haplotypes, or diplotypes in a subject can indicate that the subject is in need of continued screening for the development of AMD. Such SNPs, haplotypes, or diplotypes can be neutral SNPs, haplotypes, or diplotypes. Thus, described herein are methods identifying a subject in need of screening for the development of AMD comprising determining in the subject the identity of one or more chromosome 1 and chromosome 10 SNPs, haplotypes, or diplotypes described herein, wherein a neutral SNP, haplotype, or diplotype can indicate a subject in need of screening for the development of AMD. For example, and not to be limiting, identifying a subject with a neutral SNP, haplotype, or diplotype can indicate that the subject should be screened for the development of AMD, such as AMD, at an earlier age, and more often than would typically be done in the general population.

### C. METHODS OF IDENTIFYING APPROPRIATE AMD TREATMENTS

Described herein are methods of identifying an appropriate AMD treatment for a subject in need of treatment for AMD comprising determining in the subject the identity of one or more chromosome 1 and chromosome 10 SNPs, haplotypes, or diplotypes described herein. In one aspect, the chromosome 1 SNP can be the rs1061170 SNP in the CFH gene, wherein the genotype can be CC, CT, or TT, and the chromosome 10 SNP can be the rs10490924 SNP in the HTRA1 gene, wherein the genotype can be GG, GT, or TT. In a further aspect, the chromosome 1 haplotype can be the haplotype H1 (SEQ ID NO. 1), H2 (SEQ ID NO. 2), H3 (SEQ ID NO. 3), H4 (SEQ ID NO. 4), or H5 (SEQ ID NO. 5), the sequences of which are set forth in FIG. 11, and the chromosome 10 haplotype can include the rs 10490924 SNP in the HTRA1 gene, wherein the genotype can be GG, GT, or TT. In a further aspect, the chromosome 1 diplotype can be the diplotype H1_H1, H1_H2, H1_H3, H1_H4, H1_H5, H2_H2, H2_H3, H2_H4, H2_H5, H3_H3, H3_H4, H3_H5, H4_H4, H4_H5, or H5 H5, and the chromosome 10 diplotype can include the rs10490924 SNP in HTRA1, wherein the genotype can be GG, GT, or TT. As described herein, the presence of one or more of the chromosome 1 and chromosome 10 SNPs, haplotypes, or diplotypes disclosed herein can indicate the appropriate way to treat a subject that is in need of treatment for AMD. As used herein, "treatment" can also mean prophylactic, or preventative treatment.

In one aspect, determining the identity of certain chromosome 1 and chromosome 10 SNPs, haplotypes, or diplotypes in a subject can indicate the appropriate AMD treatment for a subject that is in need of treatment for AMD. For example, and not to be limiting, determining the identity of one or more chromosome 1 and chromosome 10 SNPs, haplotypes, or diplotypes in a subject can inform a treating physician as to the appropriate AMD treatment for the subject, that is, whether the physician should treat the subject with a complement-based therapy or a vascular-based therapy, and more specifically, whether the subject is likely to respond to or benefit from the treatment. Thus, described herein are methods for identifying an appropriate AMD treatment for a subject in need of treatment for AMD comprising determining in the subject the identity of one or more chromosome 1 and chromosome 10 SNPs, haplotypes, or diplotypes described herein, wherein the identity of the SNP, haplotype, or diplotype can indicate the appropriate AMD treatment for the subject. In one aspect, the method of identifying an appropriate AMD treatment for a subject in need of treatment can further comprise administering a therapeutic composition to the subject identified as being in need of treatment.

For example, and not to be limiting, identifying a chromosome 1 risk SNP, haplotype, or diplotype described herein, and a chromosome 10 protective or neutral SNP, haplotype, or diplotype described herein, in a subject in need of treatment for AMD, can inform a treating physician that he should treat the subject with a complement-based therapy, or that the subject will likely respond to or benefit from treatment with a complement-based therapy. Additionally, identifying a chromosome 1 protective or neutral SNP, haplotype, or diplotype described herein, and a chromosome 10 risk SNP, haplotype, or diplotype described herein, in a subject in need of treatment for AMD, can inform a treating physician that he should treat the subject with a vascular-based therapy, or that the subject is likely to respond to or benefit from treatment with a vascular-based therapy. For further example, identifying a chromosome 1 risk SNP, haplotype, or diplotype described herein, and a chromosome 10 risk SNP, haplotype, or diplotype described herein, in a subject in need of treatment for AMD, can inform a treating physician that he should treat the subject with one or more of a complement-based therapy and a vascular-based therapy, or that the subject is likely to respond to or benefit from treatment with one or more of a complement-based therapy and a vascular-based therapy. In one aspect, the treating physician can vary the order of the therapy used treat a subject having both a chromosome 1 risk SNP, haplotype, or diplotype described herein, and a chromosome 10 risk SNP, haplotype, or diplotype described herein. For example, and not to be limiting, the complement-based therapy can be administered to the subject before the vascular-based therapy. Alternatively, the vascular-based therapy can be administered before the complement-based therapy.

In another aspect, determining the identity of certain chromosome 1 and chromosome 10 SNPs, haplotypes, or diplotypes in a subject can indicate that the subject is not receiving the appropriate treatment for AMD. Thus, described herein are methods for identifying a subject not receiving the appropriate treatment for AMD comprising determining in the subject the identity of one or more chromosome 1 and chromosome 10 SNPs, haplotypes, or diplotypes described herein, wherein the identity of the SNP, haplotype, or diplotype can indicate that the subject is not receiving the appropriate treatment for AMD.

For example, and not to be limiting, identifying a chromosome 1 risk SNP, haplotype, or diplotype described herein, and a chromosome 10 protective or neutral SNP, haplotype, or diplotype described herein, in a subject in need of treatment for AMD, can inform a treating physician that he should not treat the subject with a vascular-based therapy, or that the subject is not likely to respond to or benefit from treatment with a vascular-based therapy. Additionally, identifying a chromosome 1 protective or neutral SNP, haplotype, or diplotype described herein, and a chromosome 10 risk SNP, haplotype, or diplotype described herein, in a subject in need of treatment for AMD, can inform a treating physician that he should not treat the subject with a complement-based therapy, or that the subject is not likely to respond to or benefit from treatment with a complement-based therapy. For further example, identifying a chromosome 10 risk SNP, haplotype, or diplotype described herein, in a subject in need of treatment for AMD, can inform a treating physician that he should not treat the subject with an anti-VEGF therapy, or with certain vitamin supplements, such as zinc supplements, or that the subject is not likely to respond to or benefit from treatment with an anti-VEGF therapy, or with certain vitamin supplements, such as zinc supplements.

### D. METHODS OF IDENTIFYING PATIENTS FOR CLINICAL TRIALS

Described herein are methods of identifying a subject that is appropriate for an AMD clinical trial comprising determining in the subject the identity of one or more chromosome 1 and chromosome 10 SNPs, haplotypes, or diplotypes described herein. In one aspect, the chromosome 1 SNP can be the rs1061170 SNP in the CFH gene, wherein the genotype can be CC, CT, or TT, and the chromosome 10 SNP can be the rs10490924 SNP in the HTRA1 gene, wherein the genotype can be GG, GT, or TT. In a further aspect, the chromosome 1 haplotype can be the haplotype H1 (SEQ ID NO. 1), H2 (SEQ ID NO. 2), H3 (SEQ ID NO. 3), H4 (SEQ ID NO. 4), or H5 (SEQ ID NO. 5), the sequences of which are set forth in FIG. 11, and the chromosome 10 haplotype can include the rs10490924 SNP in the HTRA1 gene, wherein the genotype can be GG, GT, or TT. In a further aspect, the chromosome 1 diplotype can be the diplotype H1_H1, H1_H2, H1_H3, H1_H4, H1_H5, H2_H2, H2_H3, H2_H4, H2_H5, H3_H3, H3_H4, H3_H5, H4_H4, H4_H5, or H5_H5, and the chromosome 10 diplotype can include the rs10490924 SNP in HTRA1, wherein the genotype can be GG, GT, or TT. As described herein, the presence of one or more of the chromosome 1 and chromosome 10 SNPs, haplotypes, or diplotypes disclosed herein can indicate whether the subject is appropriate for an AMD clinical trial. Thus, the methods described herein can be used to identify a subject that is appropriate for an age-related macular degeneration clinical trial. For example, and not to be limiting, determining the identity of certain chromosome 1 and chromosome 10 SNPs, haplotypes, or diplotypes in a subject can inform an enrolling physician as to whether the subject would be an appropriate candidate to participate in different types of clinical trials, that is, whether the subject would be appropriate for a clinical trial testing a complement-based AMD therapy or a clinical trial testing a vascular-based AMD therapy. As used herein, clinical trial means an experimental study conducted to evaluate the effectiveness and safety of a treatment or medical device by monitoring their effects on a subject or group of subjects. In one aspect, the clinical trial can be conducted to evaluate the effectiveness and safety of medications for AMD.

In one aspect, determining the identity of certain chromosome 1 and chromosome 10 SNPs, haplotypes, or diplotypes described herein in a subject can indicate that the subject is appropriate for an AMD clinical trial. Thus, described herein are methods for identifying a subject that is appropriate for an AMD clinical trial comprising determining in the subject the identity of one or more chromosome 1 and chromosome 10 SNPs, haplotypes, or diplotypes described herein, wherein the identity of the SNP, haplotype, or diplotype can indicate that the subject is appropriate for the clinical trial. In one aspect, the method of identifying a subject for the clinical trial can further comprise administering a therapeutic composition to the subject identified as being appropriate for the clinical trial. For example, a subject identified to have a chromosome 1 risk SNP, haplotype, or diplotype described herein, and a chromosome 10 protective or neutral SNP, haplotype, or diplotype described herein, can be included in a clinical trial designed to evaluate a complement-based treatment for AMD. For further example, a subject identified to have a chromosome 1 protective or neutral SNP, haplotype, or diplotype described herein, as well as a chromosome 10 risk SNP, haplotype, or diplotype described herein, can be included in a clinical trial designed to evaluate a vascular-based treatment for AMD.

In another aspect, determining the identity of certain chromosome 1 and chromosome 10 SNPs, haplotypes, or diplotypes in a subject can indicate that the subject is not appropriate for an AMD clinical trial. Thus, described herein are methods for identifying a subject that is appropriate for an AMD clinical trial comprising determining in the subject the identity of one or more chromosome 1 and chromosome 10 SNPs, haplotypes, or diplotypes described herein, wherein the identity of the SNP, haplotype, or diplotype can indicate that the subject is not appropriate for an AMD clinical trial. For example, a subject identified to have chromosome 1 and chromosome 10 protective or neutral SNPs, haplotypes, or diplotypes described herein can be excluded from participation in a clinical trial designed to evaluate certain treatments for AMD. For further example, a subject identified to have a chromosome 1 risk SNP, haplotype, or diplotype described herein, and a chromosome 10 risk SNP, haplotype, or diplotype described herein, can be excluded from participation in a clinical trial designed to evaluate a complement-based treatment for AMD.

In yet another aspect, determining the identity of certain chromosome 1 and chromosome 10 SNPs, haplotypes, or diplotypes in a subject can indicate that the subject may be appropriate for an AMD clinical trial. Thus, described herein are methods of identifying a subject appropriate for an AMD clinical trial comprising determining in the subject the identity of one or more chromosome 1 and chromosome 10 SNPs, haplotypes, or diplotypes described herein, wherein the identity of the SNP, haplotype, or diplotype can indicate a subject that may be appropriate for an AMD clinical trial.

### E. GENERAL METHODS

In methods described herein, including the methods for determining a subject's susceptibility to having or developing AMD, that comprise, in part, comprising determining in the subject the identity of one or more chromosome 1 and chromosome 10 SNPs, haplotypes, or diplotypes, the methods can optionally comprise isolating a sample from the subject. For example, disclosed herein are methods for determining a subject's susceptibility to having or developing AMD comprising isolating a sample from the subject and determining in the identity of one or more chromosome 1 and chromosome 10 SNPs, haplotypes, or diplotypes in the sample.

In some aspects of the methods described herein the methods can optionally comprise isolating or purifying a nucleic acid sample obtained from the subject.

In some aspects, the disclosed methods can utilize tissue samples from the subject to extract nucleic acids and determine the identity of SNPs, haplotypes or diplotypes. Thus, in some aspects the disclosed methods further comprise obtaining a tissue sample from the subject. "Obtaining a tissue sample" or "obtain a tissue sample" means to collect a sample of tissue either from a party having previously harvested the tissue or harvesting directly from a subject. It is understood and herein contemplated that tissue samples obtained directly from the subject can be obtained by any means known in the art including invasive and non-invasive techniques. It is also understood that methods of measurement can be direct or indirect. Examples of methods of obtaining or measuring a tissue sample can include but are not limited to venipuncture, tissue biopsy, tissue lavage, aspiration, tissue swab, spinal tap, magnetic resonance imaging (MRI), Computed Tomography (CT) scan, Positron Emission Tomography (PET) scan, and X-ray (with and without contrast media). In a further aspect, the disclosed methods can comprise purifying nucleic acid from the tissue sample obtained from the subject. Thus, in one aspect the methods disclosed herein comprise extracting nucleic acid from the tissue sample.

In some aspects of the methods described herein the methods can optionally comprise isolating or purifying a nucleic acid sample obtained from the subject and further enriching or amplifying the nucleic acid sample obtained fom the subject prior to determining in the identity of one or more chromosome 1 and chromosome 10 SNPs, haplotypes, or diplotypes in the sample. The nucleic acid sample can be enriched or purified using polymerase chain reaction (PCR), reverse transcription PCR (RT-PCR), rolling circle amplification, ligation mediated rolling circle amplification, transcription mediated amplification, ligation chain reaction, helicase-dependent amplifcation or any other known method of amplification or enrichment of nucleic acid techniques.

As described herein, the methods described herein can be used to determine proper therapuetic treatments of subject. As such, in some aspects of the methods described herein, the methods can further comprise treating or withholding treatment of the subject.

In addition, the methods described herein can also use computer implemented methods of determining in the subject the identity of one or more chromosome 1 and chromosome 10 SNPs, haplotypes, or diplotypes.

In addition, specifc primers and probes can be used to determine in the subject the identity of one or more chromosome 1 and chromosome 10 SNPs, haplotypes, or diplotypes. Examples can be found in the Examples Section and Figures of the instant application.

### F. ARRAYS

Also described herein are arrays comprising polynucleotides capable of specifically hybridizing to one or more of the SNPs, haplotypes, or diplotypes described herein. For example, and not to be limiting, described herein are arrays comprising polynucleotides capable of specifically hybridizing to the chromosome 1 SNPs and/or the chromosome 10 SNPs described herein.

Also described herein are solid supports comprising one or more polypeptides capable of specifically hybridizing to a peptide or a variant peptide encoded by the genes described herein.

Solid supports are solid-state substrates or supports with which molecules, such as analytes and analyte binding molecules, can be associated. Analytes, such as calcifying nano-particles and proteins, can be associated with solid supports directly or indirectly. For example, analytes can be directly immobilized on solid supports. Analyte capture agents, such as capture compounds, can also be immobilized on solid supports. For example, described herein are antigen binding agents capable of specifically binding to a peptide or a variant peptide encoded by the genes described herein.

A preferred form of solid support is an array. Another form of solid support is an array detector. An array detector is a solid support to which multiple different capture compounds or detection compounds have been coupled in an array, grid, or other organized pattern.

Solid-state substrates for use in solid supports can include any solid material to which molecules can be coupled. This includes materials such as acrylamide, agarose, cellulose, nitrocellulose, glass, polystyrene, polyethylene vinyl acetate, polypropylene, polymethacrylate, polyethylene, polyethylene oxide, polysilicates, polycarbonates, teflon, fluorocarbons, nylon, silicon rubber, polyanhydrides, polyglycolic acid, polylactic acid, polyorthoesters, polypropylfumerate, collagen, glycosaminoglycans, and polyamino acids. Solid-state substrates can have any useful form including thin film, membrane, bottles, dishes, fibers, woven fibers, shaped polymers, particles, beads, nanoparticles, microparticles, or a combination. Solid-state substrates and solid supports can be porous or non-porous. A preferred form for a solid-state substrate is a microtiter dish, such as a standard 96-well type. In preferred embodiments, a multiwell glass slide can be employed that normally contain one array per well. This feature allows for greater control of assay reproducibility, increased throughput and sample handling, and ease of automation.

Different compounds can be used together as a set. The set can be used as a mixture of all or subsets of the compounds used separately in separate reactions, or immobilized in an array. Compounds used separately or as mixtures can be physically separable through, for example, association with or immobilization on a solid support. An array can include a plurality of compounds immobilized at identified or predefined locations on the array. Each predefined location on the array generally can have one type of component (that is, all the components at that location are the same). Each location will have multiple copies of the component. The spatial separation of different components in the array allows separate detection and identification of the polynucleotides or polypeptides described herein.

Although preferred, it is not required that a given array be a single unit or structure. The set of compounds may be distributed over any number of solid supports. For example, at one extreme, each compound may be immobilized in a separate reaction tube or container, or on separate beads or microparticles or nanoparticles. Different modes of the disclosed method can be performed with different components (for example, different compounds specific for different proteins) immobilized on a solid support.

Some solid supports can have capture compounds, such as antibodies, attached to a solid-state substrate. Such capture compounds can be specific for calcifying nano-particles or a protein on calcifying nano-particles. Captured calcifying nano-particles or proteins can then be detected by binding of a second, detection compound, such as an antibody. The detection compound can be specific for the same or a different protein on the calcifying nano-particle.

Methods for immobilizing antibodies (and other proteins) to solid-state substrates are well established. Immobilization can be accomplished by attachment, for example, to aminated surfaces, carboxylated surfaces or hydroxylated surfaces using standard immobilization chemistries. Examples of attachment agents are cyanogen bromide, succinimide, aldehydes, tosyl chloride, avidin-biotin, photocrosslinkable agents, epoxides and maleimides. A preferred attachment agent is the heterobifunctional cross-linker N-[y-Maleimidobutyryloxy] succinimide ester (GMBS). These and other attachment agents, as well as methods for their use in attachment, are described in Protein immobilization: fundamentals and applications, Richard F. Taylor, ed. (M. Dekker, New York, 1991);, Johnstone and Thorpe, Immunochemistry In Practice (Blackwell Scientific Publications, Oxford, England, 1987) pages 209-216 and 241-242, and Immobilized Affinity Ligands; Craig T. Hermanson et al., eds. (Academic Press, New York, 1992). Antibodies can be attached to a substrate by chemically cross-linking a free amino group on the antibody to reactive side groups present within the solid-state substrate. For example, antibodies may be chemically cross-linked to a substrate that contains free amino, carboxyl, or sulfur groups using glutaraldehyde, carbodiimides, or GMBS, respectively, as cross-linker agents. In this method, aqueous solutions containing free antibodies are incubated with the solid-state substrate in the presence of glutaraldehyde or carbodiimide.

A preferred method for attaching antibodies or other proteins to a solid-state substrate is to functionalize the substrate with an amino- or thiol-silane, and then to activate the functionalized substrate with a homobifunctional cross-linker agent such as (Bis-sulfo-succinimidyl suberate (BS³) or a heterobifunctional cross-linker agent such as GMBS. For cross-linking with GMBS, glass substrates are chemically functionalized by immersing in a solution of mercaptopropyltrimethoxysilane (1% vol/vol in 95% ethanol pH 5.5) for 1 hour, rinsing in 95% ethanol and heating at 120 °C for 4 hrs. Thiol-derivatized slides are activated by immersing in a 0.5 mg/ml solution of GMBS in 1% dimethylformamide, 99% ethanol for 1 hour at room temperature. Antibodies or proteins are added directly to the activated substrate, which are then blocked with solutions containing agents such as 2% bovine serum albumin, and air-dried. Other standard immobilization chemistries are known by those of skill in the art.

Each of the components (compounds, for example) immobilized on the solid support preferably is located in a different predefined region of the solid support. Each of the different predefined regions can be physically separated from each of the other different regions. The distance between the different predefined regions of the solid support can be either fixed or variable. For example, in an array, each of the components can be arranged at fixed distances from each other, while components associated with beads will not be in a fixed spatial relationship. In particular, the use of multiple solid support units (for example, multiple beads) will result in variable distances.

Components can be associated or immobilized on a solid support at any density. Components preferably are immobilized to the solid support at a density exceeding 400 different components per cubic centimeter. Arrays of components can have any number of components. For example, an array can have at least 1,000 different components immobilized on the solid support, at least 10,000 different components immobilized on the solid support, at least 100,000 different components immobilized on the solid support, or at least 1,000,000 different components immobilized on the solid support.

Optionally, at least one address on the solid support is the sequences or part of the sequences set forth in any of the nucleic acid sequences described herein. Also disclosed are solid supports where at least one address is the sequences or portion of sequences set forth in any of the peptide sequences described herein. Solid supports can also contain at least one address is a variant of the sequences or part of the sequences set forth in any of the nucleic acid sequences described herein. Solid supports can also contain at least one address as a variant of the sequences or portion of sequences set forth in any of the peptide sequences described herein.

Also disclosed are antigen microarrays for multiplex characterization of antibody responses. For example, disclosed are antigen arrays and miniaturized antigen arrays to perform large-scale multiplex characterization of antibody responses directed against the polypeptides, polynucleotides and antibodies described herein, using submicroliter quantities of biological samples as described in Robinson et al., Autoantigen microarrays for multiplex characterization of autoantibody responses, Nat Med., 8(3):295-301 (2002), teach of constructing and using antigen arrays to perform large-scale multiplex characterization of antibody responses directed against structurally diverse antigens, using submicroliter quantities of biological samples.

Protein variants and derivatives are well understood to those of skill in the art and can involve amino acid sequence modifications. For example, amino acid sequence modifications typically fall into one or more of three classes: substitutional, insertional or deletional variants. Polypeptide variants described herein will typically exhibit at least about 70%, 75%, 80%, 85%, 90%, 91%, 92%, 93%, 94%, 95%, 96%, 97%, 98%, or 99% or more identity (determined as described below), along their length, to the polypeptide sequences set forth herein.

### G. KITS

Also described herein are kits for utilizing the methods described herein. The kits described herein can comprise an assay or assays for detecting one or more SNPs, haplotypes, or diplotypes in a nucleic acid sample of a subject, wherein the one or more SNPs, haplotypes, or diplotypes are the SNPs, haplotypes, or diplotypes described herein. In one aspect, the chromosome 1 SNP can be the rs1061170 SNP in the CFH gene, wherein the genotype can be CC, CT, or TT, and the chromosome 10 SNP can be the rs10490924 SNP in the HTRA1 gene, wherein the genotype can be GG, GT, or TT. In a further aspect, the chromosome 1 haplotype can be the haplotype H1 (SEQ ID NO. 1), H2 (SEQ ID NO. 2), H3 (SEQ ID NO. 3), H4 (SEQ ID NO. 4), or H5 (SEQ ID NO. 5), the sequences of which are set forth in FIG. 11, and the chromosome 10 haplotype can include the rs10490924 SNP in the HTRA1 gene, wherein the genotype can be GG, GT, or TT. In a further aspect, the chromosome 1 diplotype can be the diplotype H1_H1, H1_H2, H1_H3, H1_H4, H1_H5, H2_H2, H2_H3, H2_H4, H2_H5, H3_H3, H3_H4, H3_H5, H4_H4, H4_H5, or H5 H5, and the chromosome 10 diplotype can include the rs10490924 SNP in HTRA1, wherein the genotype can be GG, GT, or TT.

For example, and not to be limiting, the kits described herein can comprise an assay for detecting a SNP in a nucleic acid sample of a subject, wherein the SNP is a chromosome 1 gene or a chromosome 10 gene. In one aspect, the kits can further comprise instructions for correlating the assay results with the subject's risk for having or developing AMD. In a further aspect, the kits can further comprise instructions for correlating the assay results with the subject's appropriateness for an AMD clinical trial.

Also described herein are kits comprising one or more primers or probes for detecting a SNP, haplotype, or diplotype described herein in a nucleic acid sample of a subject. The kits described herein can comprise any of the primers or probes described herein. In one aspect, the primers and probes can be purchased from a commercial source, such as Applied Biosystems. The kits described herein can further comprise additional amplification reagents for amplifying any of the genes or nucleic acids described herein.

### H. EXAMPLES

The following examples are put forth so as to provide those of ordinary skill in the art with a complete disclosure and description of how the compounds, compositions, articles, devices and/or methods claimed herein are made and used. Unless indicated otherwise, parts are parts by weight, temperature is in °C or is at ambient temperature, and pressure is at or near atmospheric.

### 1. Genotyping Methods

In the methods described herein, all SNPs were genotyped with predesigned or custom TaqMan assays (Applied Biosystems, Foster City, California) using 10 ng of template DNA in a 5uL reaction. The thermal cycling conditions in the 384-well thermocycler (PTC-225, MJ Research) consisted of an initial hold at 95°C for 10 minutes, followed by 40 cycles of a 15-second 95°C denaturation step and a 1-minute 60°C annealing and extension step. Plates were read in a 7900HT Fast Real-Time PCR System (Applied Biosystems). Subsequently, statistical calculations were done using Microsoft Excel.

## Claims

1. A method for determining a subject's susceptibility to having or developing age-related macular degeneration (AMD),
comprising determining in the subject the identity of a chromosome 1 diplotype and a chromosome 10 diplotype,
wherein the chromosome 1 diplotype is the diplotype H1_H1, H1_H2, H1_H3, H1_H4, H1_H5, H2_H2, H2_H3, H2_H4, H2_H5, H3_H3, H3_H4, H3_H5, H4_H4, H4_H5, or H5_H5, and the chromosome 10 diplotype is the rsl0490924 SNP in HTRA1 gene, wherein the genotype in the rsl0490924 SNP is GG, GT, or TT, wherein
I) the subject is protected against having or developing AMD if the diplotype identified on chromosome 1 includes a risk haplotype and a protective haplotype, as long as the genotype at locus rsl0490924 on chromosome 10 is not the risk genotype TT, wherein the chromosome 1 risk haplotype is H1 or H2 and the chromosome 1 protective haplotype is H3 or H5; or
II) the subject has increased risk of having or developing AMD if the diplotype identified on chromosome 1 is the protective diplotype H1_H3 or H1 H5, and the genotype determined at locus rsl0490924 on chromosome 10 is the risk genotype TT; or
III) the subject has decreased risk of having or developing AMD if the diplotype identified on chromosome 1 is the protective diplotype H3_H3, H3_H4, or H3_H5, even if the genotype determined at locus rsl0490924 on chromosome 10 is the risk genotype TT.

2. The method of claim 1, further comprising:
determining whether the subject carries the genotype CC, CT, or TT at locus rsl061170 of the CFH gene on chromosome 1; and
determining the subject's susceptibility to having or developing AMD from the genotype identified in chromosome 1 and the genotype identified on chromosome 10.

3. The method of claim 2, comprising determining that the subject has an increased risk of having or developing AMD if the subject carries the risk genotype CC at locus rs1061170 on chromosome 1.

4. The method of any of claims 1 to 3,
whereby the subject is determined as being in need of treatment for AMD; or
whereby the subject is determined as being eligible for participation in a clinical trial for AMD.

5. The method of any preceding claim, wherein the haplotype, diplotype, and/or genotype is identified by amplifying or sequencing a nucleic acid sample obtained from the subject.

6. A kit for determining whether a subject has increased risk of having or developing AMD, comprising primers that are configured to specifically identify the presence of any of haplotypes H1, H2, H3, H4, and H5 (SEQ. ID NOS:1 to 5) and primers that are configured to specifically identify the genotype of the subject at rsl0490924 of chromosome 10 in a nucleic acid sample obtained from the subject, further comprising instructions for correlating the assay results with the subject's susceptibility to having or developing AMD according to the method defined in any one of claims 1 to 5.

## Patentansprüche

1. Verfahren zum Bestimmen der Anfälligkeit eines Subjekts für das Auftreten oder die Entwicklung einer altersbedingten Makuladegeneration (AMD),
umfassend die Bestimmung der Identität eines Chromosom-1-Diplotyps und eines Chromosom-10-Diplotyps in dem Subjekt,
wobei der Chromosom-1-Diplotyp der Diplotyp H1_H1, H1_H2, H1_H3, H1_H4, H1_H5, H2_H2, H2_H3, H2_H4, H2_H5, H3_H3, H3_H4, H3_H5, H4_H4, H4_H5 oder H5_H5 ist, und der Chromosom-10-Diplotyp der rs10490924-SNP im HTRA1-Gen ist,
wobei der Genotyp in dem rs10490924-SNP GG, GT oder TT ist,
wobei
I) das Subjekt gegen das Auftreten oder die Entwicklung von AMD geschützt ist, wenn der auf Chromosom 1 identifizierte Diplotyp einen Risiko-Haplotyp und einen Schutz-Haplotyp umfasst, solange der Genotyp am Locus rs10490924 auf Chromosom 10 nicht der Risiko-Genotyp TT ist, wobei der Risiko-Haplotyp auf Chromosom 1 H1 oder H2 ist und der Schutz-Haplotyp auf Chromosom 1 H3 oder H5 ist;
oder
II) das Subjekt ein erhöhtes Risiko für das Auftreten oder die Entwicklung von AMD hat, wenn der auf Chromosom 1 identifizierte Diplotyp der schützende Diplotyp H1_H3 oder H1_H5 ist und der am Locus rs10490924 auf Chromosom 10 bestimmte Genotyp der Risiko-Genotyp TT ist;
oder
III) das Subjekt ein vermindertes Risiko für das Auftreten oder die Entwicklung AMD hat, wenn der auf Chromosom 1 identifizierte Diplotyp der schützende Diplotyp H3_H3, H3_H4 oder H3_H5 ist, selbst wenn der am Locus rs10490924 auf Chromosom 10 bestimmte Genotyp der Risiko-Genotyp TT ist.

2. Verfahren gemäß Anspruch 1, weitergehend umfassend:
Bestimmen, ob das Subjekt den Genotyp CC, CT oder TT am Locus rs1061170 des CFH-Gens auf Chromosom 1 trägt; und
Bestimmen der Anfälligkeit des Subjekts für das Auftreten oder die Entwicklung von AMD, anhand des auf Chromosom 1 identifizierten Genotyps und des auf Chromosom 10 identifizierten Genotyps.

3. Verfahren gemäß Anspruch 2, umfassend Bestimmen, dass das Subjekt ein erhöhtes Risiko der Anfälligkeit für das Auftreten oder die Entwicklung von AMD hat, wenn das Subjekt den Risiko-Genotyp CC am Locus rs1061170 auf Chromosom 1 trägt.

4. Verfahren gemäß irgendeinem der Ansprüche 1 bis 3, wobei das Subjekt als behandlungsbedürftig für AMD bestimmt wird; oder
wobei das Subjekt bestimmt wird, für die Teilnahme an einer klinischen Studie für AMD in Frage zu kommen.

5. Verfahren gemäß irgendeinem der vorstehenden Ansprüche, wobei der Haplotyp, Diplotyp und/oder Genotyp durch Amplifikation oder Sequenzierung einer von dem Subjekt erhaltenen Nukleinsäureprobe identifiziert wird.

6. Kit zum Bestimmen, ob ein Subjekt ein erhöhtes Risiko der Anfälligkeit für das Auftreten oder die Entwicklung von AMD hat, umfassend Primer, die so konfiguriert sind, dass sie spezifisch das Vorhandensein eines der Haplotypen H1, H2, H3, H4 und H5 (SEQ. ID NOs: 1 bis 5) identifizieren, und Primer, die so konfiguriert sind, dass sie spezifisch den Genotyp des Subjekts an rs10490924 von Chromosom 10 in einer von dem Subjekt erhaltenen Nukleinsäureprobe identifizieren, weitergehend umfassend Anweisungen zum Korrelieren der Testergebnisse mit der Anfälligkeit des Subjekts für das Auftreten oder die Entwicklung von AMD, gemäß des in irgendeinem der Ansprüche 1 bis 5 definierten Verfahren.

## Revendications

1. Procédé de détermination de la prédisposition d'un sujet à présenter ou à développer une dégénérescence maculaire liée à l'âge (DMA),
comprenant la détermination chez le sujet de l'identité d'un diplotype sur le chromosome 1 et d'un diplotype sur le chromosome 10,
dans lequel le diplotype sur le chromosome 1 est le diplotype H1_H1, H1_H2, H1_H3, H1_H4, H1_H5, H2_H2, H2_H3, H2_H4, H2_H5, H3_H3, H3_H4, H3_H5, H4_H4, H4_H5, ou H5_H5, et le diplotype sur le chromosome 10 est le SNP rs10490924 dans le gène HTRA1,
dans lequel le génotype dans le SNP rs10490924 est GG, GT, ou TT,
dans lequel
I) le sujet est protégé contre le fait de présenter ou de développer une DMA si le diplotype identifié sur le chromosome 1 inclut un haplotype à risque et un haplotype protecteur, tant que le génotype au locus rs10490924 sur le chromosome 10 n'est pas le génotype TT à risque, dans lequel l'haplotype à risque sur le chromosome 1 est H1 ou H2 et l'haplotype protecteur sur le chromosome 1 est H3 ou H5 ; ou
II) le sujet présente un risque accru de présenter ou de développer une DMA si le diplotype identifié sur le chromosome 1 est le diplotype protecteur H1_H3 ou H1 H5, et le génotype déterminé au locus rs10490924 sur le chromosome 10 est le génotype TT à risque ; ou
III) le sujet présente un risque réduit de présenter ou de développer une DMA si le diplotype identifié sur le chromosome 1 est le diplotype protecteur H3_H3, H3_H4, ou H3_H5, même si le génotype déterminé au locus rs10490924 sur le chromosome 10 est le génotype TT à risque.

2. Procédé selon la revendication 1, comprenant en outre :
la détermination si le sujet porte le génotype CC, CT, ou TT au locus rs1061170 du gène CFH sur le chromosome 1 ; et
la détermination de la prédisposition du sujet à présenter ou à développer une DMA à partir du génotype identifié sur le chromosome 1 et du génotype identifié sur le chromosome 10.

3. Procédé selon la revendication 2, comprenant la détermination que le sujet présente un risque accru de présenter ou de développer une DMA si le sujet porte le génotype CC à risque au locus rs1061170 sur le chromosome 1.

4. Procédé selon l'une quelconque des revendications 1 à 3,
selon lequel il est déterminé que le sujet a besoin d'un traitement pour une DMA ; ou
selon lequel il est déterminé que le sujet est éligible pour participer à un essai clinique pour une DMA.

5. Procédé selon une quelconque revendication précédente, dans lequel l'haplotype, le diplotype, et/ou le génotype sont identifiés par l'amplification ou le séquençage d'un échantillon d'acide nucléique obtenu à partir du sujet.

6. Kit pour déterminer si un sujet présente un risque accru de présenter ou de développer une DMA, comprenant des amorces qui sont configurées pour identifier spécifiquement la présence de l'un quelconque des haplotypes H1, H2, H3, H4, et H5 (SEQ. ID NO : 1 à 5) et des amorces qui sont configurées pour identifier spécifiquement le génotype du sujet au niveau de rs10490924 du chromosome 10 dans un échantillon d'acide nucléique obtenu à partir du sujet,
comprenant en outre des instructions pour corréler les résultats de l'essai à la prédisposition du sujet à présenter ou à développer une DMA selon le procédé défini dans l'une quelconque des revendications 1 à 5.
